(19) Europäisches Patentamt
European Patent Office
Office européen des brevets

(11) EP 1 891 057 B1

(12) # EUROPEAN PATENT SPECIFICATION

(45) Date of publication and mention of the grant of the patent:
**27.10.2010 Bulletin 2010/43**

(21) Application number: **06743165.0**

(22) Date of filing: **13.06.2006**

(51) Int Cl.:
***C07D 403/12*** (2006.01) ***C07D 413/12*** (2006.01)
***C07D 417/12*** (2006.01) ***C07D 417/14*** (2006.01)
***C07D 413/14*** (2006.01) ***C07D 401/14*** (2006.01)
***A61K 31/435*** (2006.01) ***A61K 31/41*** (2006.01)
***A61P 25/00*** (2006.01)

(86) International application number:
**PCT/EP2006/005768**

(87) International publication number:
**WO 2006/133946 (21.12.2006 Gazette 2006/51)**

(54) **AZABICYCLO[3.1.0]HEXANE DERIVAIVES AS DOPAMIN D3 RECEPTOR MODULATORS**

AZABICYCLO[3.1.0]HEXAN-DERIVATE ALS MODULATOREN DES DOPAMIN-D3-REZEPTORS

DÉRIVÉS DE L'AZA-BICYCLO[3.1.0]HEXANE COMME MODULATEURS DU RÉCEPTEUR D3 DE LA DOPAMINE

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**HR**

(30) Priority: **14.06.2005 GB 0512099**

(43) Date of publication of application:
**27.02.2008 Bulletin 2008/09**

(73) Proprietor: **GLAXO GROUP LIMITED**
**Greenford, Middlesex UB6 0NN (GB)**

(72) Inventors:
- **ARISTA, Luca**
  **GlaxoSmithKline SpA**
  **I-37135 Verona (IT)**
- **CARDULLO, Francesca**
  **GlaxoSmithKline SpA**
  **I-37135 Verona (IT)**
- **CHECCHIA, Anna**
  **GlaxoSmithKline SpA**
  **I-37135 Verona (IT)**
- **HAMPRECHT, Dieter**
  **GlaxoSmithKline SpA**
  **I-37135 Verona (IT)**
- **MICHELI, Fabrizio**
  **GlaxoSmithKline SpA**
  **I-37135 Verona (IT)**
- **TEDESCO, Giovanna**
  **GlaxoSmithKline SpA**
  **I-37135 Verona (IT)**
- **TERRENI, Silvia**
  **GlaxoSmithKline**
  **I-37135 Verona (IT)**

(74) Representative: **Mauro, Marina Eliana et al**
**GlaxoSmithKline**
**Corporate Intellectual Property CN925.1**
**980 Great West Road**
**Brentford, Middlesex TW8 9GS (GB)**

(56) References cited:
**WO-A-97/45403** **WO-A-03/035622**
**WO-A-2005/080382**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The present invention relates to novel compounds, processes for their preparation, pharmaceutical compositions containing them and their use in therapy.

WO 2002/40471 (SmithKline Beecham) discloses certain benzazepine compounds having activity at the dopamine $D_3$ receptor.

Recently a patent application has been published as WO2005/080382 and discloses the following compounds of formula (I) or a salt thereof:

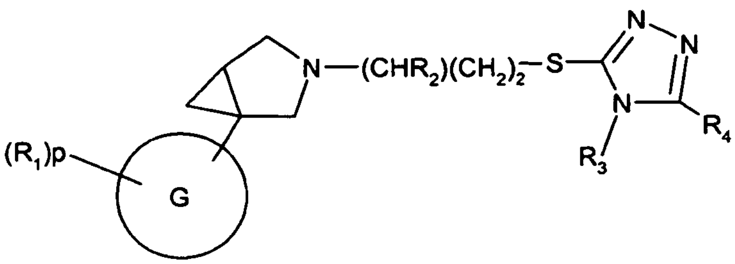

(I)

A new class of compounds which have affinity for dopamine receptors, in particular the dopamine $D_3$ receptor has been found. These compounds have potential in the treatment of conditions wherein modulation of the $D_3$ receptor is beneficial, e.g. to treat drug dependency or as antipsychotic agents.

The present invention provides a compound of formula (I) or a salt thereof:

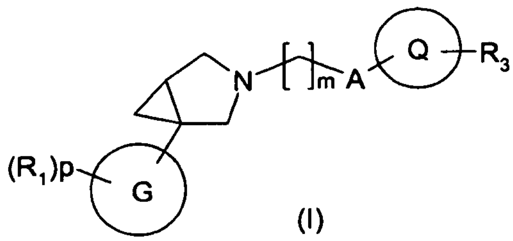

(I)

wherein

- G is selected from a group consisting of: phenyl, pyridyl, benzothiazolyl and indazolyl;
- p is 0, 1, 2, 3,4 or 5;
- $R_1$ is independently selected from a group consisting of: halogen, hydroxy, cyano, $C_1$-$_4$alkyl, halo$C_{1-4}$alkyl, $C_{1-4}$alkoxy, halo$C_{1-4}$alkoxy, $C_{1-4}$alkanoyl and $SF_5$; or corresponds to a group $R_2$;
- $R_2$ is selected from a group consisting of: isoxazolyl, -$CH_2$-N-pyrrolyl, 1,1-dioxido-2-isothiazolidinyl, thienyl, thiazolyl, pyridyl and 2-pyrrolidinonyl, and such a group is optionally substituted by one or two substituents selected from a group consisting of: halogen, cyano, $C_1$-$_4$alkyl, halo$C_{1-4}$alkyl, $C_{1-4}$alkoxy and $C_{1-4}$alkanoyl;
- m is 2, 3, 4 or 5;
- A is S, O or -$CH_2$-;
- Q is a 5-membered heteroaromatic group other than triazolyl, which 5-membered heteroaromatic group is optionally substituted by one or two $C_{1-4}$alkyl; and
- $R_3$ is hydrogen, $C_{1-4}$alkyl, phenyl, a heterocyclyl group, a 5- or 6-membered heteroaromatic group or a 8- to 11-membered bicyclic group, any of which groups is optionally substituted by 1, 2, 3 or 4 substituents selected from the group consisting of: halogen, cyano, $C_{1-4}$alkyl, halo$C_{1-4}$alkyl, $C_{1-4}$alkoxy, $C_{1-4}$alkanoyl and $SF_5$;

and when $R_1$ corresponds to $R_2$, p is 1.

The term "$C_{1-4}$alkyl" refers to an alkyl group having from one to four carbon atoms, in all isomeric forms, such as methyl, ethyl, propyl, isopropyl, butyl, isobutyl, sec-butyl and tert-butyl. The term "*n*-$C_{1-4}$alkyl" refers to the unbranched alkyls as defined above.

The term "$C_{1-4}$alkoxy" refers to a straight chain or branched chain alkoxy (or "alkyloxy") group having from one to four carbon atoms, such as methoxy, ethoxy, propoxy, isopropoxy, butoxy, isobutoxy, sec-butoxy and tert-butoxy.

The term '$C_{1-4}$ alkanoyl group' as used herein may be a linear or a branched chain alkanoyl group, for example acetyl, ethylcarbonyl, *n*-propylcarbonyl, *i*-propyl carbonyl, *n*-butylcarbonyl or *t*-butylcarbonyl and the like.

The term 'halo $C_{1-4}$ alkyl' as used herein means an alkyl group having one or more carbon atoms and wherein at least one hydrogen atom is replaced with halogen such as for example a trifluoromethyl group and the like.

The term 'halo $C_{1-4}$ alkoxy group' as used herein may be a $C_1$-$_4$ alkoxy group as defined before substituted with at least one halogen, preferably fluorine, such as $OCHF_2$, or $OCF_3$.

The term "$SF_5$" refers to pentafluorosulfanyl.

The term "halogen" and its abbreviation "halo" refer to fluorine (F), chlorine (Cl), bromine (Br) or iodine (I). Where the term "halo" is used before another group, it indicates that the group is substituted by one, two or three halogen atoms. For example, "halo$C_{1-4}$alkyl" refers to groups such as trifluoromethyl, bromoethyl, trifluoropropyl, and other groups derived from $C_{1-4}$alkyl groups as defined above; and the term "halo$C_{1-4}$alkoxy" refers to groups such as trifluoromethoxy, bromoethoxy, trifluoropropoxy, and other groups derived from $C_{1-4}$alkoxy groups as defined above.

The term "heterocyclyl" refers to a 5 or 6-membered monocyclic or 8 to 11-membered bicyclic group wherein 1, 2, 3, 4 or 5 of the carbon atoms are replaced by a heteroatom independently selected from O, S and N and which is partially or fully saturated. Examples of "heterocyclyl" which are fully saturated 5 or 6-membered monocyclic rings include pyrrolidinyl, imidazolidinyl, pyrazolidinyl, isothiazolyl, thiazolyl, tetrahydrofuranyl, dioxolanyl, piperidinyl, piperazinyl, morpholinyl, thiomorpholinyl, tetrahydrothienyl, dioxanyl, tetrahydro-2*H*-pyranyl and dithianyl. Examples of "heterocyclyl" groups which are partially saturated 5 or 6-membered monocyclic rings include oxazolinyl, isoaxazolinyl, imidazolinyl, pyrazolinyl, 1,2,3,6-tetrahydropyridyl and 3,6-dihydro-2*H*-pyranyl. Examples of "heterocyclyl" groups which are fully saturated 8 to 11-membered bicyclic rings include decahydroquinolinyl, octahydro-2*H*-1,4-benzoxazinyl and octahydro-1*H*-cyclopenta-[*b*]pyridinyl. Examples of "heterocyclyl" groups which are partially saturated 8 to 11-membered bicyclic rings include 2,3-dihydro-1*H*-indolyl, 1,2,3,4-tetrahydroquinolinyl, 1,2,3,4-tetrahydroisoquinolinyl and 2,3,4,5-tetrahydro-1*H*-3-benzazepinyl.

The term "5-membered heteroaromatic group" refers to a 5-membered aromatic group containing 1, 2, 3 or 4 heteroatoms, for example from 1 to 3 heteroatoms, selected from O, N and S. The term "5- or 6- membered heteroaromatic group" refers to a 5- or 6-membered aromatic group containing 1, 2, 3 or 4 heteroatoms, for example from 1 to 3 heteroatoms, selected from O, N and S. For example, when the group contains 2, 3 or 4 heteroatoms, one may be selected from O, N and S and the remaining heteroatoms may be N. Examples of 5-membered heteroaromatic groups include pyrrolyl, imidazolyl, pyrazolyl, oxazolyl, furazanyl, isoxazolyl, triazolyl, oxadiazolyl, isothiazolyl, thiazolyl, furyl, thienyl and thiadiazolyl. Examples of 6-membered heteroaromatic groups include pyridyl, triazinyl, pyridazinyl, pyrimidinyl and pyrazinyl.

The term "8- to 11-membered bicyclic group" refers to a bicyclic ring system containing a total of 8, 9, 10 or 11 carbon atoms, wherein 1, 2, 3 or 4 or 5 of the carbon atoms are optionally replaced by a heteroatom independently selected from O, S and N. The term includes bicyclic systems wherein both rings are aromatic, as well as bicyclic ring systems wherein one of the rings is partially or fully saturated. Examples of 8- to 11- membered bicyclic groups wherein both rings are aromatic include indenyl, naphthyl and azulenyl. Examples of 8- to 11-membered bicyclic groups having 1, 2, 3, 4 or 5 heteroatoms, in which both rings are aromatic, include: 6*H*-thieno[2,3-*b*]pyrrolyl, imidazo[2,1-b][1,3]thiazolyl, imidazo[5,1-*b*][1,3]thiazolyl, [1,3]thiazolo[3,2-*b*][1,2,4]triazolyl, indolyl, isoindolyl, indazolyl, benzimidazolyl e.g. benzimidazol-2-yl, benzoxazolyl e.g. benzoxazol-2-yl, benzisoxazolyl, benzothiazolyl, benzisothiazolyl, benzothienyl, benzofuranyl, naphthridinyl, quinolyl, quinoxalinyl, quinazolinyl, cinnolinyl and isoquinolyl. Examples of 8- to 11-membered bicyclic groups having 1, 2, 3 , 4 or 5 heteroatoms, in which one of the rings is partially or fully saturated includes dihydrobenzofuranyl, indanyl, tetrahydronaphthyl, indolinyl, isoindolinyl, tetrahydroisoquinolinyl, tetrahydroquinolyl, benzoxazinyl and benzoazepinyl.

Any of these groups may be attached to the rest of the molecule at any suitable position.

As used herein, the term "salt" refers to any salt of a compound according to the present invention prepared from an inorganic or organic acid or base, quaternary ammonium salts and internally formed salts. Physiologically acceptable salts are particularly suitable for medical applications because of their greater aqueous solubility relative to the parent compounds. Such salts must clearly have a physiologically acceptable anion or cation. Suitably physiologically acceptable salts of the compounds of the present invention include acid addition salts formed with inorganic acids such as hydrochloric, hydrobromic, hydroiodic, phosphoric, metaphosphoric, nitric and sulfuric acids, and with organic acids, such as tartaric, acetic, trifluoroacetic, citric, malic, lactic, fumaric, benzoic, formic, propionic, glycolic, gluconic, maleic, succinic, camphorsulfuric, isothionic, mucic, gentisic, isonicotinic, saccharic, glucuronic, furoic, glutamic, ascorbic, anthranilic, salicylic, phenylacetic, mandelic, embonic (pamoic), methanesulfonic, ethanesulfonic, pantothenic, stearic, sulfinilic, alginic, galacturonic and arylsulfonic, for example benzenesulfonic and p-toluenesulfonic, acids; base addition salts formed with alkali metals and alkaline earth metals and organic bases such as N,N-dibenzylethylenediamine, chloroprocaine, choline, diethanolamine, ethylenediamine, meglumaine (N-methylglucamine), lysine and procaine; and internally formed salts. Salts having a non-physiologically acceptable anion or cation are within the scope of the invention as useful intermediates for the preparation of physiologically acceptable salts and/or for use in non-therapeutic, for

example, *in vitro,* situations.

In one embodiment, G is phenyl or pyridyl.

In one embodiment, p is 1 or 2.

In another embodiment, p is 0.

In one embodiment, $R_1$ is halogen (such as chloro, bromo or fluoro), $C_{1-4}$alkoxy (such as methoxy), $C_{1-4}$alkyl (such as tert-butyl), cyano, acetyl, trifluoromethyl or trifluoromethoxy.

In one embodiment, A is sulfur.

In one embodiment, $R_2$ is a group selected from: isoxazolyl, 2-pyrrolidinonyl, 1,1-dioxido-2-isothiazolidinyl which is optionally substituted by one or two substituents selected from: halogen, cyano, $C_{1-2}$alkyl (e.g. methyl), halo$C_{1-2}$alkyl (e.g. trifluoromethyl), $C_{1-2}$alkoxy (e.g. methoxy), $C_{1-3}$alkanoyl (e.g. acetyl). For example, $R_2$ is isoxazolyl, 2-pyrrolidinonyl, $-CH_2$-N-pyrrolyl, 1,1-dioxido-2-isothiazolidinyl, 2-thienyl, 2-pyridyl or 2-thiazolyl.

In one embodiment, m is 4 or 5.

Q is a 5-membered heteroaromatic ring, which is not triazotyl. In one embodiment, the heteroaromatic ring contains at least one N.

In another embodiment, G is phenyl.

In another embodiment, p is 1.

In another embodiment, $R_1$ is trifluoromethyl

In another embodiment, m is 3.

In one embodiment, $R_3$ is hydrogen, $C_{1-4}$alkyl (such as methyl), optionally substituted phenyl (e.g. phenyl, 4-trifluoromethyl-phenyl, 3,4-difluorophenyl), an optionally substituted bicyclic group such as quinolinyl (e.g. 2-methylquinoline, 8-fluoro-2-methylquinoline), an optionally substituted pyranyl (e.g. 4-tetrahydro-2H-pyranyl), an optionally substituted pyridinyl (e.g. 3-methyl-2-pyridinyl, 2-methyl-3-pyridinyl, 3-pyridinyl, 2-methyl-6-trifluoromethyl-3-pyridinyl), an optionally substituted pyrazolyl (e.g. 5-chloro-1-methyl-1H-pyrazol-4-yl, 1-methyl-3-trifluoromethyl-1H-pyrazol-4-yl 1,5-dimethyl-1H-pyrazoly-4-yl), an optionally substituted pyrimidyl (e.g. 5-pyrimidinyl), an optionally substituted pyridazinyl (e.g. 4-pyridazinyl), an optionally substituted pyrazinyl (e.g. 5-methyl-2-pyrazinyl), an optionally substituted furanyl (e.g. 3-methyl-2-furanyl, 2,5-dimethyl-3-furanyl), an optionally substituted thienyl (e.g. 5-chloro-2-thienyl), an optionally substituted oxazolyl (e.g. 4-methyl-1,3-oxazol-5-yl, 2-methyl-5-trifluoromethyl-1,3-oxazol-4-yl), an optionally substituted isoxazolyl (e.g. 3-methyl-5-isoxazolyl), an optionally substituted thiazolyl (e.g. 2,4-dimethyl-1,3-thiazol-5-yl), an optionally substituted triazolyl (e.g. 1-methyl-1H-1,2,3-triazol-4-yl).

In one embodiment, Q is imidazolyl, thiadiazolyl, oxadiazolyl, thiazolyl, oxazolyl, triazolyl, furazanyl, isothiazolyl or isoxazolyl, each of which is optionally substituted by a $C_{1-4}$alkyl group.

In one embodiment, $R_3$ is hydrogen, $C_{1-4}$alkyl (such as methyl), optionally substituted phenyl (e.g. phenyl, 4-chlorophenyl), an optionally substituted pyridinyl (e.g. 3-pyridinyl), an optionally substituted furanyl (e.g. 2-furanyl), an optionally substituted thienyl (e.g. 5-chloro-2-thienyl).

In one embodiment, Q is imidazolyl, thiadiazolyl, oxadiazolyl, thiazolyl, oxazolyl, tetrazolyl or thienyl, each of which is optionally substituted by a $C_{1-4}$alkyl group.

In one embodiment, when $R_3$ is phenyl, a heterocyclyl group, a 5- or 6-membered heteroaromatic group or a 8- to 11-membered bicyclic group, it may be joined to the atom at the 3rd position from the bond to A, a compound of formula (IA) is provided, ie:

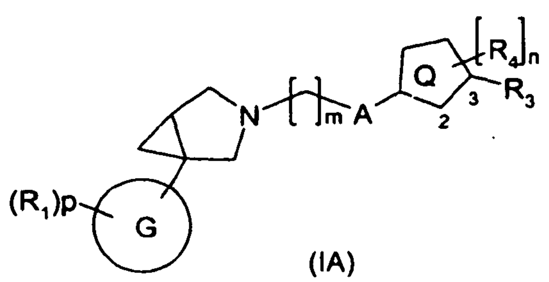

(IA)

wherein $R_1$, p, G, m, A and Q are as defined for formula (I), n is 0, 1 or 2, $R_4$ is $C_{1-4}$alkyl and $R_3$ is phenyl, a heterocyclyl group, a 5- or 6-membered heteroaromatic group or a 8-to 11-membered bicyclic group, any of which groups is optionally substituted by 1, 2, 3 or 4 substituents selected from the group consisting of: halogen, cyano, $C_{1-4}$alkyl, halo$C_{1-4}$alkyl, $C_{1-4}$alkoxy, $C_{1-4}$alkanoyl and $SF_5$.

In one embodiment, the present invention provides a compound of formula (IB) or a salt thereof:

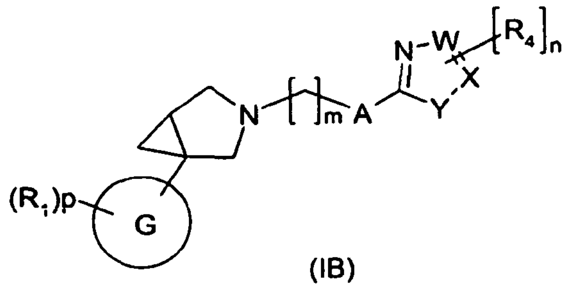

(IB)

wherein $R_1$, p, G, m and A are as defined for formula (I), n is 0, 1 or 2, $R_4$ is hydrogen or $C_{1-4}$alkyl, W is C or N, X is N or $CR_3$ and Y is O, S, NH or $NC_{1-4}$alkyl, wherein $R_3$ is hydrogen, $C_{1-4}$alkyl, phenyl, a heterocyclyl group, a 5- or 6-membered heteroaromatic group or a 8- to 11-membered bicyclic group, any of which groups is optionally substituted by 1, 2, 3 or 4 substituents selected from the group consisting of: halogen, cyano, $C_{1-4}$alkyl, halo$C_{1-4}$alkyl, $C_{1-4}$alkoxy, $C_{1-4}$alkanoyl and $SF_5$.

In one embodiment, a compound of formula (IC) or a salt thereof is provided:

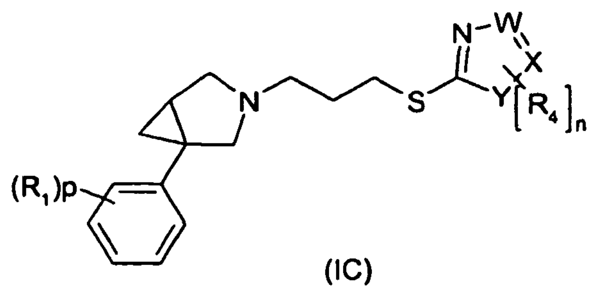

(IC)

wherein $R_1$ and p are as defined for formula (I), n is 0, 1 or 2, $R_4$ is hydrogen or $C_{1-4}$alkyl, W is C or N, X is N or $CR_3$ and Y is O, S, NH or $NC_{1-4}$alkyl, wherein $R_3$ is hydrogen, $C_{1-4}$alkyl, phenyl, a heterocyclyl group, a 5- or 6-membered heteroaromatic group or a 8- to 11-membered bicyclic group, any of which groups is optionally substituted by 1, 2, 3 or 4 substituents selected from the group consisting of: halogen, cyano, $C_{1-4}$alkyl, halo$C_{1-4}$alkyl, $C_{1-4}$alkoxy, $C_{1-4}$alkanoyl and $SF_5$.

Certain groups/substituents included in the present invention may be present as isomers. The present invention includes within its scope all such isomers, including racemates, enantiomers, tautomers and mixtures thereof. Certain of the substituted heteroaromatic groups included in compounds of formula (I) may exist in one or more tautomeric forms. The present invention includes within its scope all such tautomeric forms, including mixtures.

It will be appreciated that compounds of formula (I)' possess at least two chiral centres, namely at position 1 and 5 in the 3-azabicyclo[3.1.0]hexane portion of the molecule. Because of the presence of the fused cyclopropane compounds of formula (I) are believed to have a "*cis*" disposition of the substituents (both groups linked to the bicyclic ring system are on the same face of this bicyclic ring system). Thus, the compounds may exist in two stereoisomers which are enantiomers with respect to the chiral centres in the cyclopropane. It will also be appreciated, in common with most biologically active molecules that the level of biological activity may vary between the individual stereoisomers of a given molecule. It is intended that the scope of the invention includes all individual stereoisomers (diastereoisomers and enantiomers) and all mixtures thereof, including but not limited to racemic mixtures, which demonstrate appropriate biological activity with reference to the procedures described herein.

In another embodiment of the present invention compounds of formula (I)' are provided which correspond to the compounds of formula (I) having "cis" disposition, represented by the bold highlight of the two bonds near the cyclopropyl moiety:

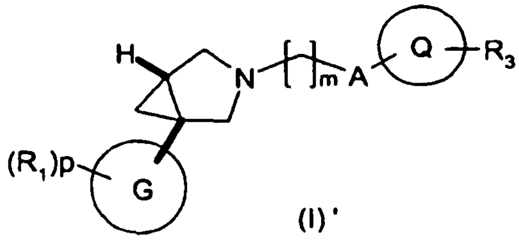

(I)'

wherein G, p, $R_1$, m, A, Q and $R_3$ are defined as above for compounds of formula (I).

In compounds of formula (I)' there are at least two chiral centres, which are located in the cyclopropane portion, as depicted below (the bold highlight of the bonds means the "cis" configuration):

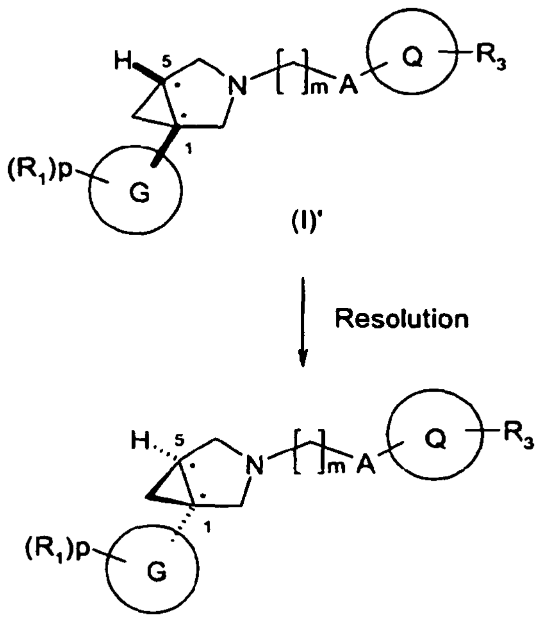


**(1S, 5R configuration)**

It should be noted that, when G is one of the heteroaromatic group described, the configuration of compounds of formula (IA) may become (1R, 5R) due to different Cahn-Ingold-Prelog nomenclature priorities.

In a further embodiment of the present invention, there is provided a compound of formula (ID) or a salt thereof that correspond to a stereochemical isomer of a compound of formula (I), enriched in configuration (1S,5R) (or possibly (1R,5R) when G is one of the heteroaromatic group described):

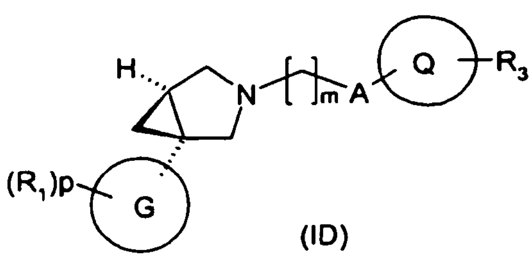

(ID)

wherein G, p, $R_1$, m, A, Q and $R_3$ are defined as above for compounds of formula (I)'.

It is intended in the context of the present invention that stereochemical isomers enriched in configuration (1S,5R) (or possibly (1R,5R) when G is one of the heteroaromatic group described) of formula (I) correspond in one embodiment to at least 90% enatiomeric excess. In another embodiment the isomers correspond to at least 95% enantiomeric excess. In another embodiment the isomers correspond to at least 99% enantiomeric excess.

The strategy for determining the absolute configuration of the compounds of the present invention comprised as a first step the preparation of the chiral intermediate, (1*S*,5*R*)-1-[4-(trifluoromethyl)phenyl]-3-azabicyclo[3.1.0]hexane:

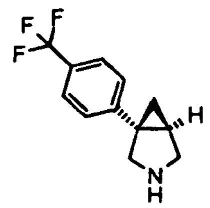

by using (S)-(+) acetyl mandelic acid as resolving agent.

In the literature the absolute configuration of a series of compounds similar to this chiral intermediate is known, see J. Med Chem 1981, 24(5), 481-90. For some compounds disclosed in the reference the absolute configuration was proved by single crystal X-ray analysis.

The assignment of the absolute configuration of the chiral intermediate, (1*S*,5*R*)-1-[4-(trifluorometh yl)phenyl]-3-azabicyclo[3.1.0]hexane, was confirmed by a single crystal X-ray structure obtained from a crystal of (1S,SR)-1-[4-(trifluoromethyl)phenyl]-3-azabicyclo[3.1.0]hexane, (S)-(+)-mandelic acid salt. Both the analysis based on the known configuration of the (S)-(+)-mandelic acid and on the basis of anomalous dispersion effects confirmed the assignment of this compound as being (1S,5R)-1-[4-(trifluoromethyl)phenyl]-3-azabicyclo[3.1.0]hexane. As this chiral intermediate was used for the preparation of all the example compounds of the present invention, the absolute configurations of all the example compounds were believed to be the same as the chiral intermediate, based on a reasonable assumption by a skilled person in the art.

Chiral molecules exhibit vibrational circular dichroism (VCD). Vibrational circular dichroism (VCD) is the differential interaction of a chiral molecule with left and right circularly polarized infrared radiation during vibrational excitation.

The VCD spectrum of a chiral molecule is dependent on its three-dimensional structure. Most importantly, the VCD spectrum of a chiral molecule is a sensitive function of its absolute configuration and, in the case of flexible molecules, of its conformation. In principle, therefore, VCD permits the determination of the structure of a chiral molecule. VCD spectra were first measured in the 1970s. Subsequently, VCD instrumentation has developed enormously in spectral range and in sensitivity. Currently, VCD spectra of liquids and solutions can be measured over the majority of the fundamental infrared (IR) spectral range (ν≥ 650 cm-1) with high sensitivity at acceptable resolution (1-5 cm-1) using both dispersive and Fourier Transform (FT) VCD instrumentation. Very recently, commercial FT VCD instrumentation has become available, greatly enhancing the accessibility of VCD spectra.

The use of VCD as a reliable method for the determination of absolute configuration of chiral molecules is now well established (see for example Shah RD, et al., Curr Opin Drug Disc Dev 2001;4:764-774; Freedman TB, et al., Helv Chim Acta 2002; 85:1160-1165; Dyatkin AB, et al. Chirality 2002;14:215-219; Solladie -Cavallo A, Balaz Met al., Tetrahedron Assym 2001;12:2605-2611; Nafie LA, et al. Circular dichroism, principles and applications, 2nd ed. New York: John Wiley & Sons; 2000. p 97-131; Nafie LA, et al. in: Yan B, Gremlish H-U, editors. Infrared and Raman spectroscopy of biological materials. New York: Marcel Dekker; 2001. p 15-54; Polavarapu PL, et al., J Anal Chem 2000;366:727-734; Stephens PJ, et al., Chirality 2000;12:172-179; Solladie' -Cavallo A, et al., Eur J Org Chem 2002: 1788-1796).

The method entails comparison of observed IR and VCD spectra with calculations of the spectra for a specific configuration and provides information both on the absolute configuration and on the solution conformation.

Given an experimental spectrum of a chiral molecule whose absolute configuration and/or conformation are unknown and to be determined, the general procedure is as follows: 1) all possible structures are defined; 2) the spectra of these structures are predicted; and 3) predicted spectra are compared to the experimental spectrum. The correct structure will give a spectrum in agreement with experiment; incorrect structures will give spectra in disagreement with experiment.

VCD spectra are always measured simultaneously with vibrational unpolarized absorption spectra ("infrared (IR) spectra") and the two vibrational spectra together provide more information than does the VCD spectrum alone. In addition, vibrational unpolarized absorption spectra are automatically predicted simultaneously with VCD spectra.

For ab initio assignments, VCD and unpolarized IR spectra were calculated using the Gaussian 98 software package.

When chiral organic molecules are synthesized (or, if natural products, isolated) their optical rotations are routinely measured at one frequency or at a small number of discrete frequencies in the visible-near ultraviolet spectral

region. Most commonly, the specific rotation at one frequency, that of the sodium D line, $[\alpha]_D$, is measured. The frequencies used lie below the threshold for electronic absorption, i.e., they are in the "transparent" spectral region. Optical rotation is a reflection of the enantiomeric excess (ee) of the sample and of the absolute configuration (AC) of the predominant enantiomer.

When the optical rotation at a given frequency for 100% ee is available, the measured optical rotation at the same frequency enables the sample ee to be determined. The determination of ee is the predominant application of discrete frequency, transparent spectral region optical rotations. In principle, the AC of the predominant enantiomer, if unknown, can also be determined. However, the determination of AC from optical rotation requires an algorithm which reliably predicts the optical rotations of molecules of known AC and a number of methodologies have been proposed for predicting discrete frequency, transparent spectral region optical rotations (Eliel EL, Wilen SH. Stereochemistry of organic compounds. New York: John Wiley & Sons; 1994. Chapter 13).

Very recently, developments in ab initio Density Functional Theory (DFT) have radically improved the accuracy of optical rotation calculation. As a result, for the first time it has become possible to routinely obtain ACs from optical rotations.

For ab initio OR assignments, the Dalton Quantum Chemistry Program was used.

In one embodiment, a stereochemical isomer enriched in the (1S,5R) configuration of formula (IE) (or possibly (1R,5R) when G is one of the heteroaromatic group described) or a salt thereof is provided:

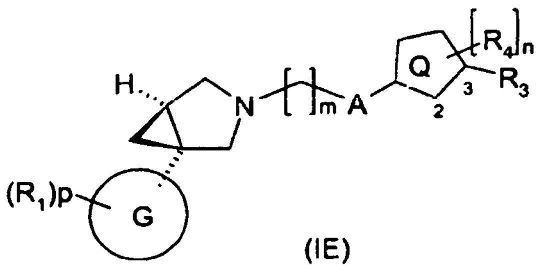

(IE)

wherein $R_1$, p, G, m, A and Q are as defined for formula (I), n is 0, 1 or 2, $R_4$ is $C_{1-4}$alkyl and $R_3$ is phenyl, a heterocyclyl group, a 5- or 6-membered heteroaromatic group or a 8-to 11-membered bicyclic group, any of which groups is optionally substituted by 1, 2, 3 or 4 substituents selected from the group consisting of: halogen, cyano, $C_{1-4}$alkyl, halo$C_{1-4}$alkyl, $C_{1-4}$alkoxy, $C_{1-4}$alkanoyl and $SF_5$.

In one embodiment, a stereochemical isomer enriched in the (1S,5R) configuration of formula (IF) (or possibly (1R,5R) when G is one of the heteroaromatic group described) or a salt thereof is provided:

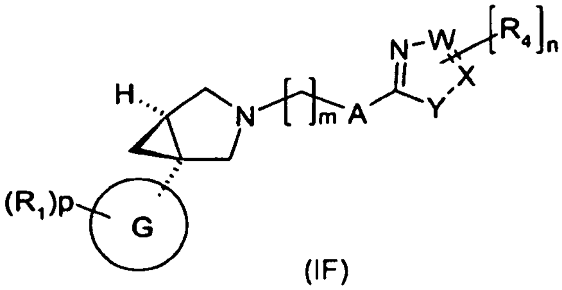

(IF)

wherein $R_1$, p, G, m and A are as defined for formula (I), n is 0, 1 or 2, $R_4$ is hydrogen or $C_{1-4}$alkyl, W is C or N, X is N or $CR_3$ and Y is O, S, NH or $NC_{1-4}$alkyl, wherein $R_3$ is hydrogen, $C_{1-4}$alkyl, phenyl, a heterocyclyl group, a 5- or 6-membered heteroaromatic group or a 8- to 11-membered bicyclic group, any of which groups is optionally substituted by 1, 2, 3 or 4 substituents selected from the group consisting of: halogen, cyano, $C_{1-4}$alkyl, halo$C_{1-4}$alkyl, $C_{1-4}$alkoxy, $C_{1-4}$alkanoyl and $SF_5$.

In one embodiment, a stereochemical isomer enriched in the (1S,5R) configuration of formula (IG) or a salt thereof is provided:

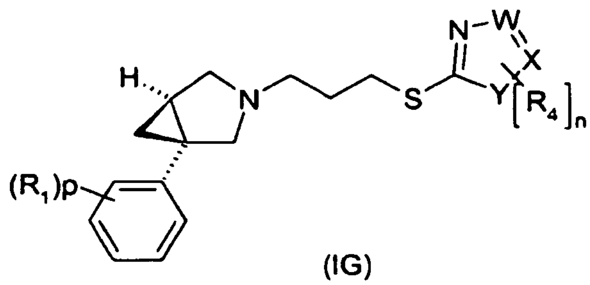

(IG)

wherein $R_1$ and p are as defined for formula (I), n is 0, 1 or 2, $R_4$ is hydrogen or $C_{1-4}$alkyl, W is C or N, X is N or $CR_3$ and Y is O, S, NH or $NC_{1-4}$alkyl, wherein $R_3$ is hydrogen, $C_{1-4}$alkyl, phenyl, a heterocyclyl group, a 5- or 6-membered heteroaromatic group or a 8- to 11-membered bicyclic group, any of which groups is optionally substituted by 1, 2, 3 or 4 substituents selected from the group consisting of: halogen, cyano, $C_{1-4}$alkyl, halo$C_{1-4}$alkyl, $C_{1-4}$alkoxy, $C_{1-4}$alkanoyl and $SF_5$.

It will be appreciated that for use in medicine the salts of the compounds of the invention should be pharmaceutically (i.e physiologically) acceptable. Suitable pharmaceutically acceptable salts will be apparent to those skilled in the art and include for example acid addition salts formed with inorganic acids e.g. hydrochloric, hydrobromic, sulfuric, nitric or phosphoric acid; and organic acids e.g. succinic, maleic, acetic, fumaric, citric, tartaric, benzoic, p-toluenesulfonic, methanesulfonic or naphthalenesulfonic acid. Other non-pharmaceutically acceptable salts eg. oxalates, may be used, for example in the isolation of compounds of the invention and are included within the scope of this invention. Also included within the scope of the invention are solvates, hydrates, complexes and prodrugs of compounds of the invention. Pharmaceutical acceptable salts may also be prepared from other salts, including other salts, of the compound of formula (I) using conventional methods.

Certain of the compounds of the invention may form acid addition salts with one or more equivalents of the acid. The present invention includes within its scope all possible stoichiometric and non-stoichiometric forms. Certain of the compounds of the invention may form acid addition salts with less than one equivalent of the acid, or one or more equivalents of the acid. The present invention includes within its scope all possible stoichiometric and non-stoichiometric forms.

Those skilled in the art of organic chemistry will appreciate that many organic compounds can form complexes with solvents in which they are reacted or from which they are precipitated or crystallized. These complexes are known as "solvates". For example, a complex with water is known as a "hydrate". Solvates of the compound of the invention are within the scope of the invention. The compounds of formula (I) may readily be isolated in association with solvent molecules by crystallisation or evaporation of an appropriate solvent to give the corresponding solvates.

Those skilled in the art will appreciate that in the preparation of the compound of the invention or a solvate thereof it may be necessary and/or desirable to protect one or more sensitive groups in the molecule to prevent undesirable side reactions. Suitable protecting groups for use according to the present invention are well known to those skilled in the art and may be used in a conventional manner. See, for example, "Protective groups in organic synthesis" by T.W. Greene and P.G.M. Wuts (John Wiley & sons 1991) or "Protecting Groups" by P.J. Kocienski (Georg Thieme Verlag 1994). Examples of suitable amino protecting groups include acyl type protecting groups (e.g. formyl, trifluoroacetyl, acetyl), aromatic urethane type protecting groups (e.g. benzyloxycarbonyl (Cbz) and substituted Cbz), aliphatic urethane protecting groups (e.g. 9-fluorenylmethoxycarbonyl (Fmoc), t-butyloxycarbonyl (Boc), isopropyloxycarbonyl, cyclohexyloxycarbonyl) and alkyl type protecting groups (e.g. benzyl, trityl, chlorotrityl). Examples of suitable oxygen protecting groups may include for example alky silyl groups, such as trimethylsilyl or tertbutyldimethylsilyl; alkyl ethers such as tetrahydropyranyl or tert-butyl; or esters such as acetate.

When a specific enantiomer of a compound of general formula (I) is required, this may be obtained for example by resolution of a corresponding enantiomeric mixture of a compound of formula (I) using conventional methods. Thus the required enantiomer may be obtained from the racemic compound of formula (I) by use of chiral HPLC procedure.

In one embodiment of the present invention compounds are provided having a molecular weight of 800 or less. In another embodiment compounds are provided having a molecular weight of 600 or less. Generally, and without being limited thereto, such compounds may have higher oral bioavailability, and sometimes higher solubility and/or brain penetrancy. Molecular weight here refers to that of the unsolvated free base compound, excluding any molecular weight contributed by addition salts, solvent (e.g. water) molecules, prodrug molecular parts cleaved off *in vivo*, etc.

In general, the compounds or salts of the invention should be interpreted as excluding those compounds (if any) which are so chemically unstable, either per se or in water, that they are clearly unsuitable for pharmaceutical use through all administration routes, whether oral, parenteral or otherwise. Such compounds are known to the skilled

chemist. Prodrugs or compounds which are stable *ex vivo* and which are convertable in the mammalian (e.g. human) body to the inventive compounds are however included.

Example compounds of the present invention include:

(1S,5R)-3-{3-[(1-methyl-1H-tetrazol-5-yl)thio]propyl}-1-[4-(trifluoromethyl)phenyl]-3-azabicyclo[3.1.0]hexane;
(1S,5R)-3-{3-[(1-methyl-1H-imidazol-2-yl)thio]propyl}-1-[4-(trifluoromethyl)phenyl]-3-azabicyclo[3.1.0]hexane;
(1S,5R)-3-[3-(1H-imidazol-2-ylthio)propyl]-1-[4-(trifluoromethyl)phenyl]-3-azabicyclo[3.1.0]hexane;
(1S,5R)-3-{3-[(4,5-dimethyl-1H-imidazol-2-yl)thio]propyl}-1-[4-(trifluoromethyl)phenyl]-3-azabicyclo[3.1.0]hexane;
(1S,5R)-3-{3-[(5-methyl-1,3,4-thiadiazol-2-yl)thio]propyl}-1-[4-(trifluoromethyl)phenyl]-3-azabicyclo[3.1.0]hexane;
(1S,5R)-3-{3-[(5-methyl-1,3,4-oxadiazol-2-yl)thio]propyl}-1-[4-(trifluoromethyl)phenyl]-3-azabicyclo[3.1.0]hexane;
(1S,5R)-3-{3-[(4,5-dimethyl-1,3-oxazol-2-yl)thio]propyl}-1-[4-(trifluoromethyl)phenyl]-3-azabicyclo[3.1.0]hexane;
(1S,5R)-3-[3-(1,3-oxazol-2-ylthio)propyl]-1-[4-(trifluoromethyl)phenyl]-3-azabicyclo[3.1.0]hexane;
(1S,5R)-3-[3-(1,3-thiazol-2-ylthio)propyl]-1-[4-(trifluoromethyl)phenyl]-3-azabicyclo[3.1.0]hexane;
(1S,5R)- 3-(3-{[5-(3-pyridinyl)-1,3-thiazol-2-yl]thio}propyl)-1-[4-(trifluoromethyl)phenyl]-3-azabicyclo[3.1.0]hexane;
(11S,5R)-3-(3-{[1-methyl-5-(3-pyridinyl)-1H-imidazol-2-yl]thio}propyl)-1-[4-(trifluoromethyl)phenyl]-3-azabicyclo [3.1.0]hexane;
(1S,5R)-3-{3-[(1-methyl-5-phenyl-1H-imidazol-2-yl)thio]propyl}-1-[4-(trifluoromethyl)phenyl]-3-azabicyclo[3.1.0] hexane;
(1S,5R)-3-{3-[(5-phenyl-1,3-thiazol-2-yl)thio]propyl}-1-[4-(trifluoromethyl)phenyl]-3-azabicyclo[3.1.0]hexane;
(1S,5R)-3-{3-[(5-phenyl-1,3,4-thiadiazol-2-yl)thio]propyl}-1-[4-(trifluoromethyl)phenyl]-3-azabicyclo[3.1.0]hexane;
(1S,5R)-3-(3-{[5-(5-chloro-2-thienyl)-1,3,4-thiadiazol-2-yl]thio}propyl)-1-[4-(trifluoromethyl)phenyl]-3-azabicyclo [3.1.0]hexane;
(1S,5R)-3-(3-{[5-(2-pyridinyl)-1,3,4-oxadiazol-2-yl]thio}propyl)-1-[4-(trifluoromethyl)phenyl]-3-azabicyclo[3.1.0] hexane;
(1S,5R)-3-(3-{[5-(4-chlorophenyl)-1-methyl-1H-imidazol-2-yl]thio}propyl)-1-[4-(trifluoromethyl)phenyl]-3-azabicyclo [3.1.0]hexane;
(1S,5R)-3-(3-{[5-(2-furanyl)-1,3,4-oxadiazol-2-yl]thio}propyl)-1-[4-(trifluoromethyl)phenyl]-3-azabicyclo[3.1.0]hexane;
(1S,5R)-3-(3-{[5-(4-chlorophenyl)-1,3,4-oxadiazol-2-yl]thio}propyl)-1-[4-(trifluoromethyl)phenyl]-3-azabicyclo [3.1.0]hexane;
(1S,5R)-3-(3-{[4-(4-chlorophenyl)-1,3-thiazol-2-yl]thio}propyl)-1-[4-(trifluoromethyl)phenyl]-3-azabicyclo[3.1.0]hexane;
(1S,5R)-3-{3-[(4-phenyl-1H-imidazol-2-yl)thio]propyl}-1-[4-(trifluoromethyl)phenyl]-3-azabicyclo[3.1.0]hexane;

and salts thereof.

The present invention also provides a process for preparing a compound of formula (I) or a salt thereof as defined above, the process comprising:

(a) reacting a compound of formula (II):

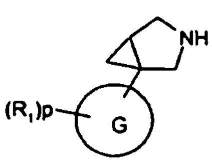

(II)

wherein $R_1$ and p are as defined for formula (I), with a compound of formula (III):

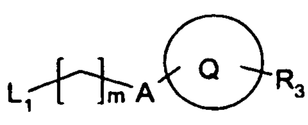

(III)

wherein m, Q and $R_4$ are as defined for formula (I) and $L_1$ is a leaving group, or

(b) for a compound of formula (I) wherein p is 1 or 2, reacting a compound of formula (IV):

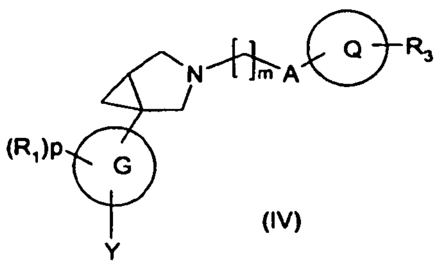

(IV)

wherein $R_1$, m, A, Q and $R_3$ are as defined for formula (I), p is 0 or 1 and Y is halogen, a perfluoroalkylsulfonyloxy group (e.g. trifluoromethylsulfonyloxy), or Y is a group M selected from a boron derivative (e.g. a boronic acid function $B(OH)_2$) or a metal function such as trialkylstannyl (e.g. $SnBu_3$), zinc halide or magnesium halide; with a compound R1-Y1, wherein Y1 is halogen when Y is a group M; or when Y is halogen or a perfluoroalkylsulfonyloxy group Y1 is a group M as defined above or hydrogen that can be activated by a suitable base (e.g. $Cs_2CO_3$) in the presence of a suitable transition metal (e.g. Pd); "leaving group" is as understood by a skilled chemist, i.e. a group which can be displaced by a nucleophile in e.g. a $S_N2$, $S_N1$ or $S_NAr$ type reaction; or

(c) for a compound of formula (I) wherein A is S, reacting a compound of formula (V):

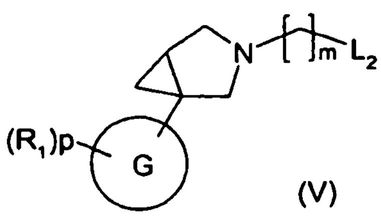

(V)

wherein $R_1$, p, G and m are as defined for formula (I) and $L_2$ is a leaving group, with a compound of formula (VI):

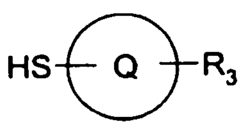

(VI)

wherein Q and $R_3$ are as defined for formula (I); or

(d) for a compound of formula (I) wherein A is O, reacting a compound of formula (VII):

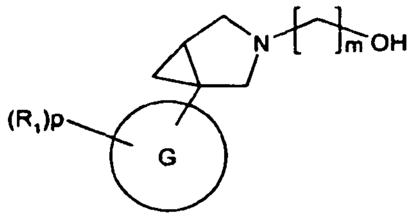

(VII)

wherein G, $R_1$, p and m are as defined for formula (I), with a compound of formula (VIII):

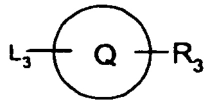

(VIII)

wherein Q and $R_3$ are as defined for formula (I) and $L_3$ is a leaving group;
or

(e) for a compound of formula (I) wherein A is $CH_2$, reacting a compound of formula (IX):

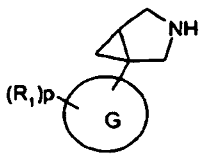

(IX)

wherein G, $R_1$ and p are as defined for formula (I), with a compound of formula (X):

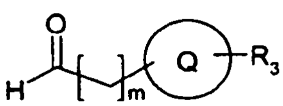

(X)

wherein $R_2$, $R_3$ and $R_4$ are as defined for formula (I);
and thereafter optionally, for process (a), (b), (c), (d) or (e):

(i) removing any protecting group(s); and/or
(ii) forming a salt; and/or
(iii) converting a compound of formula (I) or a salt thereof to another compound of formula (I) or a salt thereof.

Process (a) may be performed using conventional methods for the formation of a tertiary amine. The leaving group $L_1$ can be halogen such as chlorine. Alternatively $L_1$ can be a sulfonyloxy group such $C_{1-4}$alkylsulfonyloxy (e.g. methanesulfonyloxy), $C_{1-4}$alkylsulfonyloxy or halo$C_{1-4}$alkylsulfonyloxy (e.g. trifluoromethanesulfonyloxy); or arylsulfonyloxy wherein aryl is optionally substituted phenyl, an optionally substituted 5- or 6- membered heteroaromatic group, or an optionally substituted bicyclic group, for example optionally substituted phenyl, wherein in each case the optional substituents are one or more $C_{1-2}$alkyl groups; e.g. *para*-toluenesulfonyloxy. When $L_1$ is a halogen the reaction may be carried out using a base such as potassium carbonate in the presence of a source of iodide such as sodium iodide in

a solvent such as *N*,*N*-dimethylformamide at a suitable temperature, e.g. 60 °C.

Compounds of formula (II) may be prepared by methods well known in the art (e.g. J. Med. Chem. 1981, 24, 481-490 or WO2005/080382). Interconversion of groups $R_1$ may be effected by methodology well known in the art (e.g. demethylation of a methoxy group resulting in a hydroxy group using a suitable Lewis acidic reagent such as boron tribromide in an inert solvent such as dichloromethane). sence of a suitable protecting group for the secondary amine, such as N-trifluoroacetyl.

Compounds of formula (III) may be prepared by methods well known in the art.

Compounds of formula (V) may be prepared by methods well known in the art starting from compounds of formula (II).

Compounds of formula (VI) may be prepared by methods well known in the art or may be commercially available.

Alternatively compounds of formula (VIa), i.e. compounds of formula (VI) wherein Q is imidazolyl or thiazolyl and $R_3$ is 3-pyridinyl, may be prepared starting from commercially available according to the following synthetic Scheme 1:

<u>Scheme 1</u>

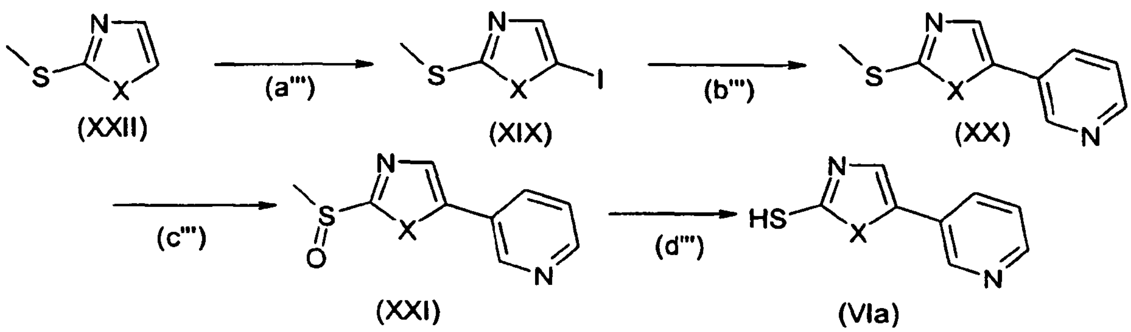

(XXII) (XIX) (XX) (XXI) (VIa)

wherein X represents NMe or S;
and wherein

- step (a''') means iodination of compounds of formula (XXII) to obtain compounds of formula (XIX);
- step (b''') means Suzuki Coupling performed on compounds of formula (XIX) to obtain compounds of formula (XX);
- step (c''') means oxidation of compounds of formula (XX) to obtain the corresponding sulfoxide of formula (XXI);
- step (d''') means conversion of compounds of formula (XXI) to obtain sulphydril compounds of formula (VIa).

Step (a''') may be conveniantly performed by treatment with $I_2$ in DMSO at 0°C.

Step (b''') may be conveniently performed by refluxing a mixture of a compound of formula (XIX), $Pd(OAc)_2$, $PPh_3$, $Na_2CO_3$ and pyridine-3-boronic acid in PrOH for 6 hours.

Step (c''') may be conveniently performed by treatment of compounds of formula (XX) with a solution of MCPBA.

Step (d''') may be conveniently performed treatment of compounds of formula (XXI) with trifluoroacetic anhydride at reflux for 6 hours.

Reaction of a compound of formula (IV) with R1-Y1 according to process (b) may be effected in the presence of a transition metal e.g., palladium catalyst such as *bis*-triphenylphosphinepalladium dichloride, *tetrakis*-triphenylphosphinepalladium (0) or the complex formed *in situ* from tris(dibenzylideneacetone) dipalladium(0) and 4,5-bis(diphenylphosphino)-9,9-dimethylxanthene. When M is a boronic acid function such as $B(OH)_2$ the reaction may be carried out under basic conditions, for example using aqueous sodium carbonate in a suitable solvent such as dioxane. When M is trialkylstannyl the reaction may be carried out in an inert solvent, such as xylene or dioxane optionally in the presence of LiCl. When M is a zinc or magnesium halide the reaction may be effected in an aprotic solvent such as tetrahydrofuran. When M is hydrogen that can be activated by a suitable base (e.g. $Cs_2CO_3$) in the presence of a suitable transition metal (e.g. Pd) the reaction may be carried out in an inert solvent such as dioxane in the presence of a suitable base such as $Cs_2CO_3$. The substituent Y may be halogen such as bromine, or a sulfonyloxy group such as trifluoromethylsulfonyloxy; and Y1 is may be a group M, such as hydrogen that can be activated by a suitable base (e.g. $Cs_2CO_3$) in the presence of a suitable transition metal (e.g. Pd).

In one aspect of the present invention, there is provided a synthetic process for the preparation of compounds of formula (II) wherein G is phenyl. The process may be conveniently performed also for preparing compounds of formula (IIa), in which the phenyl moiety is replaced by pyridine. This process comprises the following steps:

(a')

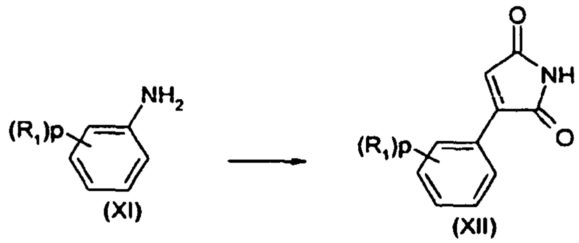

(XI) (XII)

(b')

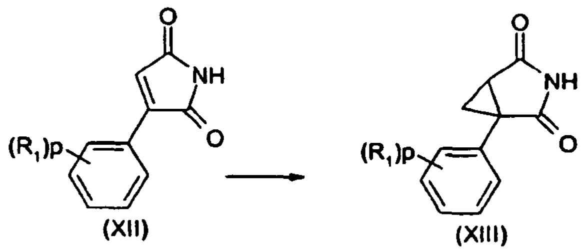

(XII) (XIII)

(c')

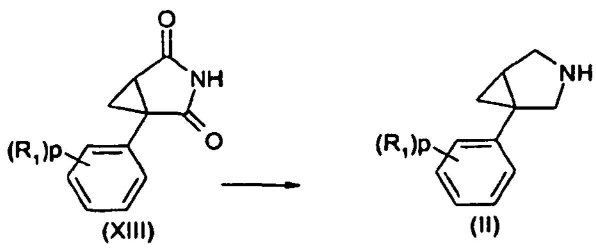

(XIII) (II)

wherein:

step(a') means diazotation of an aniline (XI) followed by reaction with maleimide to give 3-arylmaleimide (XII);
step (b') means cycloropanation of (XII) to provide bicyclic imide (XIII);
step (c') means reduction of imide (XIII) to give compounds of formula (II).

Step (a') may be effected using conventional methods for the Meerwein reaction (e.g. J. Am. Chem. Soc. 1955, 77, 2313 describes the formation of arylmaleimides using this approach). Alternatively, in many cases this step is suitably performed applying a procedure where to a mixture of maleimide, an appropriate copper (II) salt such as anhydrous $CuCl_2$, and a suitable organonitrite, such as *tert*-butyl nitrite, in a compatible solvent, such as acetonitrile, is slowly added a solution of a compound of formula (XI). This is followed by allowing time to react as appropriate and a suitable workup.

Step (b') consists of slow addition of a solution of purified compound of formula (XII), or mixtures containing a compound of formula (XII), dissolved in a suitable solvent such as dimethylsulfoxide, to a solution of trimethylsulfoxonium iodide in a suitable solvent such as dimethylsulfoxide and a suitable base, such as sodium hydride. This is followed by allowing time to react as appropriate and a suitable workup.

Step (c') can be performed using a suitable reducing agent in a compatible solvent, such as borane in tetrahydrofuran or Red-Al® in toluene at an appropriate temperature, such as for example 65 °C in the case of borane as the reducing agent. This is followed by a suitable workup.

In another aspect of the present invention an alternative synthetic process for the preparation of compounds of formula (II) is provided, comprising the following steps:

(a")

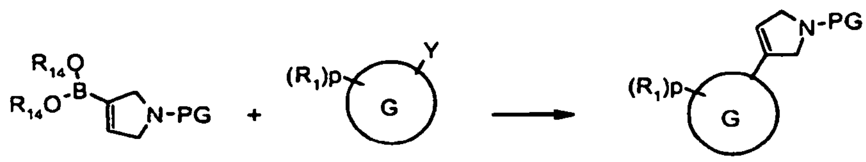

(XIV) (XV) (XVI)

(b")

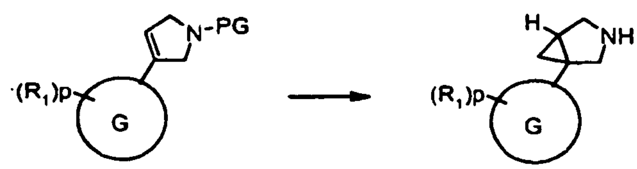

(XVI) (II)

wherein:

$R_1$, p and G are as defined for formula (I), $R_{14}O$ is a suitable alkoxy group, PG is an appropriate protecting group and Y may be halogen such as bromine, or a sulfonyloxy group such as trifluoromethylsulfonyloxy;
wherein

step (a") means coupling reaction of a (2,5-dihydro-1H-pyrrol-3-yl)boronate (XIV) with the aromatic halogen or sulfonyloxy derivative (XV);
step (b") means cycloropanation of (XVI) followed by, if appropriate, deprotection to provide bicyclic amine (II).

Step (a") may be effected using conventional methods for the Suzuki coupling, e.g. using tetrakis(triphenylphosphine)palladium(0) as the source of catalytic palladium(0) in the presence of cesium fluoride in an appropriate solvent such as tetrahydrofuran at a suitable temperature. $(R_{14}O)_2B$ may suitably be 4,4,5,5-tetramethyl-1,3,2-dioxaborolan-2-yl and PG benzyl, representing a compound of structure (X) as reported in Synlett 2002, 5, 829-831.
Step (b") consists of a cyclopropanation reaction effected for example using the reagent generated from trimethylsulfoxonium iodide and a suitable base such as sodium hydride, in a compatible solvent, for example dimethylsulfoxide. This is followed by a deprotection reaction.
In process (d), compounds of formula (VII) may be prepared by methods well known in the art (e.g. J. Med. Chem. 1981, 24, 481-490). For example, compounds of formula (VII) may be made by reacting a compound of formula (II) as defined above with a compound of formula (XVIII):

$L_3$-$(CH_2)$m-O-PG (XVIII)

wherein m is as defined for formula (I), $L_3$ is a leaving group such as bromine, and PG is a protecting group such as demethylethyldimethylsilane, followed by an appropriate deprotection procedure.
Interconversion reactions between compounds of formula (I) and salts thereof may be performed using methods well known in the art. Examples include:

(i) converting one or more of $R_1$ from alkoxy (e.g.methoxy) to hydroxy,
(ii) converting one or more of $R_1$ from hydroxy to sulfonyloxy, such as alkylsulfonyloxy or haloalkylsulfonyloxy, e.g. methanesulfonyloxy or alkylsulfonyloxy or trifluoromethanesulfonyloxy,
(iii) converting one or more of $R_1$ from halogen or perfluoroalkylsulfonyloxy to cyano;

and optionally thereafter forming a salt of formula (I).
Compounds of formula (I) have been found to exhibit affinity for dopamine receptors, in particular the $D_3$ receptor, and are expected to be useful in the treatment of disease states which require modulation of such receptors, such as psychotic conditions. Such affinity is typically calculated from the $IC_{50}$ as the concentration of a compound necessary to displace 50% of the radiolabeled ligand from the receptor, and is reported as a "$K_i$" value calculated by

the following equation:

$$K_i = \frac{IC_{50}}{1 + L / K_D}$$

where L = radioligand and $K_D$ = affinity of radioligand for receptor (Cheng and Prusoff, Biochem. Pharmacol. 22:3099, 1973).

In the context of the present invention pKi (corresponding to the antilogarithm of Ki) is used instead of Ki and the compounds of the present invention typically show pKi greater than 7. In one aspect the present invention provides compounds of formula (I) having a pKi comprised between 7 and 8. In another aspect the present invention provides compounds of formula (I) having a pKi between 8 and 9. In a further aspect the present invention provides compounds of formula (I) having a pKi greater than 9.

Many of the compounds of formula (I) have also been found to have greater affinity for dopamine $D_3$ than for $D_2$ receptors. The therapeutic effect of currently available antipsychotic agents (neuroleptics) is generally believed to be exerted via blockade of $D_2$ receptors; however this mechanism is also thought to be responsible for undesirable extrapyramidal side effects (eps) associated with many neuroleptic agents. It has been suggested that blockade of the recently characterised dopamine $D_3$ receptor may give rise to beneficial antipsychotic activity without significant eps. (see for example Sokoloff et al, Nature, 1990; 347: 146-151; and Schwartz et al, Clinical Neuropharmacology, Vol 16, No.4, 295-314, 1993). In one embodiment compounds of the present invention are provided which have higher (e.g. ≥10x or ≥100x higher) affinity for dopamine $D_3$ than dopamine $D_2$ receptors (such affinity can be measured using standard methodology for example using cloned dopamine receptors - see herein). Said compounds may suitably be used as selective modulators of $D_3$ receptors.

From the localisation of $D_3$ receptors, it could also be envisaged that the compounds could also have utility for the treatment of substance abuse where it has been suggested that $D_3$ receptors are involved (e.g. see Levant, 1997, Pharmacol. Rev., 49, 231-252). Examples of such substance abuse include alcohol, cocaine, heroin and nicotine abuse. Other conditions which may be treated by the compounds include dyskinetic disorders such as Parkinson's disease, neuroleptic-induced parkinsonism and tardive dyskinesias; depression; anxiety, cognitive impairment including memory disorders such as Alzheimers disease, sexual dysfunction, sleep disorders, emesis, movement disorders, amnesia, aggression, autism, vertigo, dementia, circadian rhythm disorders and gastric motility disorders e.g. IBS.
Other conditions which may be treated with the compounds of the invention include obsessive compulsive (OC) spectrum disorders as below defined.

Compounds of formula (I) may be used for treatment of all aspects of drug dependency including withdrawal symptoms from drugs of abuse such as alcohol, cocaine, opiates, nicotine, benzodiazepines and inhibition of tolerance induced by opioids. In addition, compounds of formula (I) and pharmaceutically acceptable salts and solvates thereof may be used to reduce craving and therefore will be useful in the treatment of drug craving. Drug craving can be defined as the incentive motivation to self-administer a psychoactive substance that was previously consumed. Three main factors are involved in the development and maintenance of drug craving: (1) Dysphoric states during drug withdrawal can function as a negative reinforcer leading to craving; (2) Environmental stimuli associated with drug effects can become progressively more powerful (sensitization) in controlling drug seeking or craving, and (3) A cognition (memory) of the ability of drugs to promote pleasurable effects and to alleviate a dysphoric state during withdrawal. Craving may account for the difficulty that individuals have in giving up drugs of abuse and therefore contributes significantly to the development and maintenance of drug dependence.

The compounds of formula (I) are of potential use as antipsychotic agents for example in the treatment of schizophrenia, schizo-affective disorders, psychotic depression, mania, paranoid and delusional disorders. Furthermore, they could have utility as adjunct therapy in Parkinsons Disease, particularly with compounds such as L-DOPA and possibly dopaminergic agonists, to reduce the side effects experienced with these treatments on long term use (e.g. see Schwartz et al., Brain Res. Reviews, 1998, 26, 236-242).

Compounds of formula (I) may be used for the treatment of obsessive compulsive disorders (OCD) and of psychiatric and neurospychiatric disorders related to them (OC spectrum disorders).

Compounds of formula (I) may be useful in the treatment of sexual dysfunction, such as premature ejaculation.

Compounds of formula (I) may be useful for the treatment of cognition impairment.

Within the context of the present invention, the terms describing the indications used herein are classified in the Diagnostic and Statistical Manual of Mental Disorders, 4th Edition, published by the American Psychiatric Association (DSM-IV) and/or the International Classification of Diseases, 10th Edition (ICD-10). The various subtypes of the disorders mentioned herein are contemplated as part of the present invention. Numbers in brackets after the listed diseases below refer to the classification code in DSM-IV.

Within the context of the present invention, the term "psychotic disorder" includes :-Schizophrenia including

the subtypes Paranoid Type (295.30), Disorganised Type (295.10), Catatonic Type (295.20), Undifferentiated Type (295.90) and Residual Type (295.60); Schizophreniform Disorder (295.40); Schizoaffective Disorder (295.70) including the subtypes Bipolar Type and Depressive Type; Delusional Disorder (297.1) including the subtypes Erotomanic Type, Grandiose Type, Jealous Type, Persecutory Type, Somatic Type, Mixed Type and Unspecified Type; Brief Psychotic Disorder (298.8); Shared Psychotic Disorder (297.3); Psychotic Disorder Due to a General Medical Condition including the subtypes With Delusions and With Hallucinations; Substance-Induced Psychotic Disorder including the subtypes With Delusions (293.81) and With Hallucinations (293.82); and Psychotic Disorder Not Otherwise Specified (298.9).

Within the context of the present invention, the term "substance-related disorder" includes:-

Substance-related disorders including Substance Use Disorders such as Substance Dependence, Substance Craving and Substance Abuse; Substance-Induced Disorders such as Substance Intoxication, Substance Withdrawal, Substance-Induced Delirium, Substance-Induced Persisting Dementia, Substance-Induced Persisting Amnestic Disorder, Substance-Induced Psychotic Disorder, Substance-Induced Mood Disorder, Substance-Induced Anxiety Disorder, Substance-Induced Sexual Dysfunction, Substance-Induced Sleep Disorder and Hallucinogen Persisting Perception Disorder (Flashbacks); Alcohol-Related Disorders such as Alcohol Dependence (303.90), Alcohol Abuse (305.00), Alcohol Intoxication (303.00), Alcohol Withdrawal (291.81), Alcohol Intoxication Delirium, Alcohol Withdrawal Delirium, Alcohol-Induced Persisting Dementia, Alcohol-Induced Persisting Amnestic Disorder, Alcohol-Induced Psychotic Disorder, Alcohol-Induced Mood Disorder, Alcohol-Induced Anxiety Disorder, Alcohol-Induced Sexual Dysfunction, Alcohol-Induced Sleep Disorder and Alcohol-Related Disorder Not Otherwise Specified (291.9); Amphetamine (or Amphetamine-Like)-Related Disorders such as Amphetamine Dependence (304.40), Amphetamine Abuse (305.70), Amphetamine Intoxication (292.89), Amphetamine Withdrawal (292.0), Amphetamine Intoxication Delirium, Amphetamine Induced Psychotic Disorder, Amphetamine-Induced Mood Disorder, Amphetamine-Induced Anxiety Disorder, Amphetamine-Induced Sexual Dysfunction, Amphetamine-Induced Sleep Disorder and Amphetamine-Related Disorder Not Otherwise Specified (292.9); Caffeine Related Disorders such as Caffeine Intoxication (305.90), Caffeine-Induced Anxiety Disorder, Caffeine-Induced Sleep Disorder and Caffeine-Related Disorder Not Otherwise Specified (292.9); Cannabis-Related Disorders such as Cannabis Dependence (304.30), Cannabis Abuse (305.20), Cannabis Intoxication (292.89), Cannabis Intoxication Delirium, Cannabis-Induced Psychotic Disorder, Cannabis-Induced Anxiety Disorder and Cannabis-Related Disorder Not Otherwise Specified (292.9); Cocaine-Related Disorders such as Cocaine Dependence (304.20), Cocaine Abuse (305.60), Cocaine Intoxication (292.89), Cocaine Withdrawal (292.0), Cocaine Intoxication Delirium, Cocaine-Induced Psychotic Disorder, Cocaine-Induced Mood Disorder, Cocaine-Induced Anxiety Disorder, Cocaine-Induced Sexual Dysfunction, Cocaine-Induced Sleep Disorder and Cocaine-Related Disorder Not Otherwise Specified (292.9); Hallucinogen-Related Disorders such as Hallucinogen Dependence (304.50), Hallucinogen Abuse (305.30), Hallucinogen Intoxication (292.89), Hallucinogen Persisting Perception Disorder (Flashbacks) (292.89), Hallucinogen Intoxication Delirium, Hallucinogen-Induced Psychotic Disorder, Hallucinogen-Induced Mood Disorder, Hallucinogen-Induced Anxiety Disorder and Hallucinogen-Related Disorder Not Otherwise Specified (292.9); Inhalant-Related Disorders such as Inhalant Dependence (304.60), Inhalant Abuse (305.90), Inhalant Intoxication (292.89), Inhalant Intoxication Delirium, Inhalant-Induced Persisting Dementia, Inhalant-Induced Psychotic Disorder, Inhalant-Induced Mood Disorder, Inhalant-Induced Anxiety Disorder and Inhalant-Related Disorder Not Otherwise Specified (292.9); Nicotine-Related Disorders such as Nicotine Dependence (305.1), Nicotine Withdrawal (292.0) and Nicotine-Related Disorder Not Otherwise Specified (292.9); Opioid-Related Disorders such as Opioid Dependence (304.00), Opioid Abuse (305.50), Opioid Intoxication (292.89), Opioid Withdrawal (292.0), Opioid Intoxication Delirium, Opioid-Induced Psychotic Disorder, Opioid-Induced Mood Disorder, Opioid-Induced Sexual Dysfunction, Opioid-Induced Sleep Disorder and Opioid-Related Disorder Not Otherwise Specified (292.9); Phencyclidine (or Phencyclidine-t_ike)-Related Disorders such as Phencyclidine Dependence (304.60), Phencyclidine Abuse (305.90), Phencyclidine Intoxication (292.89), Phencyclidine Intoxication Delirium, Phencyclidine-Induced Psychotic Disorder, Phencyclidine-Induced Mood Disorder, Phencyclidine-Induced Anxiety Disorder and Phencyclidine-Related Disorder Not Otherwise Specified (292.9); Sedative-, Hypnotic-, or Anxiolytic-Related Disorders such as Sedative, Hypnotic, or Anxiolytic Dependence (304.10), Sedative, Hypnotic, or Anxiolytic Abuse (305.40), Sedative, Hypnotic, or Anxiolytic Intoxication (292.89), Sedative, Hypnotic, or Anxiolytic Withdrawal (292.0), Sedative, Hypnotic, or Anxiolytic Intoxication Delirium, Sedative, Hypnotic, or Anxiolytic Withdrawal Delirium, Sedative-, Hypnotic-, or Anxiolytic-Persisting Dementia, Sedative-, Hypnotic-, or Anxiolytic-Persisting Amnestic Disorder, Sedative-, Hypnotic-, or Anxiolytic-Induced Psychotic Disorder, Sedative-, Hypnotic-, or Anxiolytic-Induced Mood Disorder, Sedative-, Hypnotic-, or Anxiolytic-Induced Anxiety Disorder Sedative-, Hypnotic-, or Anxiolytic-Induced Sexual Dysfunction, Sedative-, Hypnotic-, or Anxiolytic-Induced Sleep Disorder and Sedative-, Hypnotic-, or Anxiolytic-Related Disorder Not Otherwise Specified (292.9); Polysubstance-Related Disorder such as Polysubstance Dependence (304.80); and Other (or Unknown) Substance-Related Disorders such as Anabolic Steroids, Nitrate Inhalants and Nitrous Oxide.

Within the context of the present invention, the term "obsessive compulsive spectrum disorder" includes:

Obsessive compulsive disorders (300.3), somatoform disorders including body dysmorphic disorder (300.7) and hyperchondriasis (300.7), bulimia nervosa (307.51), anorexia nervosa (307.1), eating disorders not elsewhere classified (307.50) such as binge eating, impulse control disorders not elsewhere classified (including intermitted explosive disorder (312.34), compulsive buying or shopping, repetitive self-mutilation, onychophagia, psychogenic excoriation, kleptomania (312.32), pathological gambling (312.31), trichotillomania (312.39) and internet addiction), paraphilia (302.70) and nonparaphilic sexual addictions, Sydeham's chorea, torticollis, autistic disorders (299.0), compulsive hoarding, and movement disorders, including Tourette's syndrome (307.23).

Within the context of the present invention, the term "sexual dysfunction" includes also premature ejaculation (302.75).

Within the present invention the term "cognition impairment" includes cognition impairment in other diseases such as schizophrenia, bipolar disorder, depression, other psychiatric disorders and psychotic conditions associated with cognitive impairment, e.g. Alzheimer's disease.

The invention also provides a compound of formula (I) or a pharmaceutically acceptable salt thereof for use in therapy.

The invention also provides a compound of formula (I) or a pharmaceutically acceptable salt thereof for use in the treatment of a condition in a mammal for which modulation [especially inhibition/antagonism (which may also translate into inverse agonism in constitutively active receptor systems)] of dopamine receptors (especially dopamine $D_3$ receptors) is beneficial..

The invention also provides the use of a compound of formula (I) or a pharmaceutically acceptable salt thereof in the manufacture of a medicament for the treatment of a condition in a mammal for which modulation [especially inhibition/antagonism (which may also translate into inverse agonism in constitutively active receptor systems)] of dopamine receptors (especially dopamine $D_3$ receptors) is beneficial.

In one embodiment, $D_3$ antagonists according to the present invention are used in the treatment of psychoses such as schizophrenia, in the treatment of substance abuse, in the treatment of obsessive compulsive spectrum disorders, in the treatment of sexual dysfunction and in the treatment of cognition impairment.

Also provided is the use of a compound of formula (I) or a pharmaceutically acceptable salt thereof in the manufacture of a medicament for the treatment of a psychotic condition (e.g. schizophrenia), substance abuse in a mammal, obsessive compulsive spectrum disorders, sexual dysfunctions and cognition impairment.

Also provided is a compound of formula (I) or a pharmaceutically acceptable salt thereof for use in the treatment of a psychotic condition (e.g. schizophrenia), substance abuse, obsessive compulsive spectrum disorders, sexual dysfunction and cognition impairment in a mammal.

Also provided is a compound of formula (I) or a pharmaceutically acceptable salt thereof for use as an active therapeutic substance in a mammal, e.g. for use in the treatment of any of the conditions described herein.

"Treatment" includes prophylaxis, where this is appropriate for the relevant condition(s).

For use in medicine, the compounds of the present invention are usually administered as a standard pharmaceutical composition. The present invention therefore provides in a further aspect a pharmaceutical composition comprising a compound of formula (I) or a pharmaceutically (i.e physiologically) acceptable salt thereof and a pharmaceutically (i.e physiologically) acceptable carrier. The pharmaceutical composition can be for use in the treatment of any of the conditions described herein.

The compounds of formula (I) may be administered by any convenient method, for example by oral, parenteral (e.g. intravenous), buccal, sublingual, nasal, rectal or transdermal administration and the pharmaceutical compositions adapted accordingly.

The compounds of formula (I) and their salts which are active when given orally can be formulated as liquids or solids, for example syrups, suspensions or emulsions, tablets, capsules and lozenges.

A liquid formulation will generally consist of a suspension or solution of the compound or salt in a suitable liquid carrier(s) for example an aqueous solvent such as water, ethanol or glycerine, or a non-aqueous solvent, such as polyethylene glycol or an oil. The formulation may also contain a suspending agent, preservative, flavouring or colouring agent.

A composition in the form of a tablet can be prepared using any suitable pharmaceutical carrier(s) routinely used for preparing solid formulations. Examples of such carriers include magnesium stearate, starch, lactose, sucrose and cellulose.

A composition in the form of a capsule can be prepared using routine encapsulation procedures. For example, pellets containing the active ingredient can be prepared using standard carriers and then filled into a hard gelatin capsule; alternatively, a dispersion or suspension can be prepared using any suitable pharmaceutical carrier(s), for example aqueous gums, celluloses, silicates or oils and the dispersion or suspension then filled into a soft gelatin capsule.

Typical parenteral compositions consist of a solution or suspension of the compound or salt in a sterile aqueous carrier or parenterally acceptable oil, for example polyethylene glycol, polyvinyl pyrrolidone, lecithin, arachis oil or sesame oil. Alternatively, the solution can be lyophilised and then reconstituted with a suitable solvent just prior to administration.

Compositions for nasal administration may conveniently be formulated as aerosols, drops, gels and powders.

Aerosol formulations typically comprise a solution or fine suspension of the active substance in a pharmaceutically acceptable aqueous or non-aqueous solvent and are usually presented in single or multidose quantities in sterile form in a sealed container, which can take the form of a cartridge or refill for use with an atomising device. Alternatively the sealed container may be a unitary dispensing device such as a single dose nasal inhaler or an aerosol dispenser fitted with a metering valve which is intended for disposal once the contents of the container have been exhausted. Where the dosage form comprises an aerosol dispenser, it will contain a propellant which can be a compressed gas such as compressed air or an organic propellant such as a fluoro-chlorohydrocarbon. The aerosol dosage forms can also take the form of a pump-atomiser.

Compositions suitable for buccal or sublingual administration include tablets, lozenges and pastilles, wherein the active ingredient is formulated with a carrier such as sugar and acacia, tragacanth, or gelatin and glycerin.

Compositions for rectal administration are conveniently in the form of suppositories containing a conventional suppository base such as cocoa butter.

Compositions suitable for transdermal administration include ointments, gels and patches.

In one embodiment, the composition is in unit dose form such as a tablet, capsule or ampoule.

Each dosage unit for oral administration contains for example from 1 to 250 mg (and for parenteral administration contains for example from 0.1 to 25 mg) of a compound of the formula (I) or a salt thereof calculated as the free base.

The pharmaceutically acceptable compounds of the invention will normally be administered in a daily dosage regimen (for an adult patient) of, for example, an oral dose of between 1 mg and 500 mg, for example between 10 mg and 400 mg, e.g. between 10 and 250 mg or an intravenous, subcutaneous, or intramuscular dose of between 0.1 mg and 100 mg, for example between 0.1 mg and 50 mg, e.g. between 1 and 25 mg of the compound of the formula (I) or a salt thereof calculated as the free base, the compound being administered 1 to 4 times per day. Suitably the compounds will be administered for a period of continuous therapy, for example for a week or more.

**Biological Test Methods**

Functional potency and intrinsic activity of compounds of this invention can be measured by the following GTPγS scintillation proximity assay (GTPγS-SPA). Cells used in the study are Chinese Hamster Ovary (CHO) Cells.
Cell Line
CHO_D2
CHO_D3

Compounds may be tested according to two alternative protocols:

a) Cell membranes are prepared as follows. Cell pellets are resuspended in 10 volumes of 50mM HEPES, 1 mM EDTA pH 7.4, using KOH. On the day the following proteases are added to the buffer just prior to giving the homogenisation buffer.
$10^{-6}$M Leupeptin (Sigma L2884) - 5000 x stock = 5 mg/ml in buffer
25ug/ml Bacitracin (Sigma B0125) - 1000 x stock = 25 mg/ml in buffer
1 mM PMSF - 1000 x stock = 17 mg/ml in 100% ethanol
$2x10^{-6}$M Pepstain A - 1000 x stock = 2 mM in 100% DMSO

The cells are homogenised by 2 x 15 second bursts in a 1 litre Glass Waring blender in a class two biohazard cabinet. The resulting suspension is spun at 500g for 20 mins (Beckman T21 centrifuge: 1550 rpm). The supernatant is withdrawn with a 25 ml pipette, aliquotted into pre-chilled centrifuge tubes and spun at 48,000g to pellet membrane fragments (Beckman T1270: 23,000 rpm for 30mins). The final 48,000g pellet is resuspended in Homogenisation Buffer, (4 x the volume of the original cell pellet). The 48,000g pellet is resuspended by vortexing for 5 seconds and homogenized in a dounce homogenizer 10-15 stokes. The prep is distributed into appropriate sized aliquots, (200-1000ul), in polypropylene tubes and store at -80° C. Protein content in the membrane preparations is evaluated with the Bradford protein assay.

The final top concentration of test drug is 3uM in the assay and 11 points serial dilution curves 1:4 in 100% DMSO are carried out using a Biomek FX. The test drug at 1% total assay volume (TAV) is added to a solid, white, 384 well assay plate. 50%TAV of precoupled (for 90 mins at 4°C) membranes, 5μg/well, and Wheatgerm Agglutinin Polystyrene Scintillation Proximity Assay beads (RPNQ0260, Amersham), 0.25mg/well, in 20mM HEPES pH 7.4, 100mM NaCl, 10mM $MgCl_2$, 60μg/ml saponin and 30μM GDP is added. The third addition was a 20% TAV addition of either buffer, (agonist format) or $EC_{80}$ final assay concentration of agonist, Quinelorane, prepared in assay buffer (antagonist format). The assay was started by the addition of 29%TAV of GTPγ[$^{35}$S] 0.38nM final (37MBq/ml, 1160Ci/mmol, Amersham). After all additions assay plates are spun down for 1 min at 1,000rpm. Assay plates are counted on a Viewlux, 613/55 filter, for 5 min., between 2-6 hours after the final addition.

The effect of the test drug over the basal generates $EC_{50}$ value by an iterative least squares curve fitting

programme, expressed in the table as $pEC_{50}$ (i.e. $-logEC_{50}$). The ratio between the maximal effect of the test drug and the maximal effect of full agonist, Quinelorane, generates the Intrinsic Activity (IA) value (i.e. IA = 1 full agonist, IA < 1 partial agonist). fpKi values of test drug are calculated from the $IC_{50}$ generated by "antagonist format" experiment, using Cheng & Prusoff equation: fKi = $IC_{50}$ / 1+([A] / $EC_{50}$) where: [A] is the concentration of the agonist 5-HT in the assay and $EC_{50}$ is the 5-HT $EC_{50}$ value obtained in the same experiment. fpKi is defined as -logfKi.

b) Cell membranes are prepared as follows. Cell pellets are resuspended in 10 volumes of 50mM HEPES, 1mM EDTA pH 7.4, using KOH. On the day the following proteases are added to the buffer just prior to giving the homogenisation buffer.

$10^{-4}$M Leupeptin (Sigma L2884) - 5000 x stock = 5 mg/ml in buffer
25ug/ml Bacitracin (Sigma B0125) - 1000 x stock = 25 mg/ml in buffer
1mM PMSF - 1000 x stock = 17 mg/ml in 100% ethanol
$2x10^{-6}$M Pepstain A - 1000 x stock = 2 mM in 100% DMSO

The cells were homgenised within a glass waring blender for 2 x 15 secs in 200mls of 50mM HEPES + 10-4M leupeptin + 25ug/ml bacitracin + 1 mM EDTA + 1 mM PMSF + 2uM Pepstatin A, (the latter 2 reagents added as fresh x100 and x 500 stocks respectively in ethanol). The blender was plunged into ice for 5 mins after the first burst and 10-40 mins after the final burst to allow foam to dissipate. The material was then spun at 500g for 20 mins and the supernatant spun for 36 mins at 48,000g. The pellet was resuspended in the same buffer as above but without PMSF and Pepstatin A. The material was then forced through a 0.6mm needle, made up to the required volume, (usually x4 the volume of the original cell pellet), aliquoted and stored frozen at -80 deg C.

The final top concentration of test drug is 3uM in the assay and 11 points serial dilution curves 1:4 in 100% DMSO are carried out using a Biomek FX. The test drug at 1% total assay volume (TAV) is added to a solid, white, 384 well assay plate. 50%TAV of precoupled (for 60 mins at RT) membranes, 5μg/well, and Wheatgerm Agglutinin Polystyrene Scintillation Proximity Assay beads (RPNQ0260, Amersham), 0.25mg/well, in 20mM HEPES pH 7.4, 100mM NaCl, 10mM MgCl2, 60μg/ml saponin and 30□M GDP is added. The third addition was a 20% TAV addition of either buffer, (agonist format) or EC80 final assay concentration of agonist, Quinelorane, prepared in assay buffer (antagonist format). The assay was started by the addition of 29%TAV of GTP[35S] 0.38nM final (37MBq/ml, 1160Ci/mmol, Amersham). After all additions assay plates are spun down for 1 min at 1,000rpm. Assay plates are counted on a Viewlux, 613/55 filter, for 5 min., between 3-6 hours after the final addition.

The effect of the test drug over the basal generates EC50 value by an iterative least squares curve fitting programme, expressed in the table as pEC50 (i.e. $-logEC_{50}$). The ratio between the maximal effect of the test drug and the maximal effect of full agonist, Quinelorane, generates the Intrinsic Activity (IA) value (i.e. IA = 1 full agonist, IA < 1 partial agonist). fpKi values of test drug are calculated from the $IC_{50}$ generated by "antagonist format" experiment, using Cheng & Prusoff equation: fKi = IC50 / 1+([A] / EC50) where: [A] is the concentration of the agonist Quinelorane in the assay and EC50 is the Quinelorane EC50 value obtained in the same experiment. fpKi is defined as -logfKi.

The compounds of the invention listed above have pKi values within the range of 7.0-10.5 at the dopamine D3 receptor. pKi results are only estimated to be accurate to about ±0.3-0.5.

The compounds of the invention listed above have a selectivity over D2 greater than 30.

**Examples**

The invention is further illustrated by the following non-limiting examples.

All temperatures refer to °C. Infrared spectra were measured on a FT-IR instrument. Compounds were analysed by direct infusion of the sample dissolved in acetonitrile into a mass spectra operated in positive electro spray (ES+) ionisation mode. Proton Magnetic Resonance ($^1$H-NMR) spectra were recorded at 400 MHz, chemical shifts are reported in ppm downfield (d) from $Me_4Si$, used as internal standard, and are assigned as singlets (s), broad singlets (bs), doublets (d), doublets of doublets (dd), triplets (t), quartets (q) or multiplets (m).

Experimental vibrational circular dichroism (VCD) spectra were measured using a ChirallRTM VCD spectrometer operating in the 2000-800 cm-1 frequency range. Spectra were measured at room temperature (23o C) using a sealed transmission cell with barium fluoride windows and a path length of 100 microns. (Scan times varied from 60 to 120 minutes per isomer.) Sample solutions were typically prepared by dissolving 10 milligrams of each enantiomer in 100 microliters of deutero-chloroform ($CDCl_3$). For ab initio assignments, VCD and unpolarized IR spectra were calculated using the Gaussian 98 software package. 1.

Optical rotations were measured using a (Perkin Elmer Model 241) polarimeter operating at 589 nm (Sodium source). Measurements were made using a 1 decimeter microcell thermostated at 23o C. Concentrations were typically 10 mg/ml (c=0.01). For ab initio OR assignments, the Dalton Quantum Chemistry Program was used.

Column chromathography was carried out over silica gel (Merck AG Darmstaadt, Germany). The following abbreviations are used in the text: AcOEt = ethyl acetate, $Et_2O$ = dietyl ether, TFAA = trifluoroacetic acid anhydride, DMSO = dimethylsulfoxide, MCPBA = metachloroperbenzoic acid, SCX = strong cation exchanger, Tlc refers to thin

layer chromatography on silica plates, and dried refers to a solution dried over anhydrous sodium sulphate, r.t. (RT) refers to room temperature, Rt = retention time.

**Preparation 1: 3-[4-(trifluoromethyl)phenyl]-1*H*-pyrrole-2,5-dione**

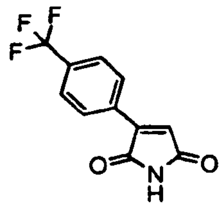

A mixture of hydrochloric acid (37%, 285 mL) and water (190 mL) was added to 4-(trifluoromethyl)aniline (150 g, 116 mL) at room temperature with vigorous stirring and the formed precipitate was allowed to stir for further 30 minutes. Temperature was reduced to 0 ˚C and sodium nitrite (70.6 g) in 180 mL of water was added dropwise to the stirred suspension. At the end of diazotisation, a clear yellow solution was obtained. Maleimide (180 g) in acetone (1.1 l) was added dropwise at 0 ˚C and then the pH of the solution was adjusted to 3-3.5 by adding sodium acetate. Copper (II) chloride (18.8 g) was added to the vigorously stirred mixture. After a few minutes a gas started to develop (conspicuous foaming). The reaction mixture was allowed to stir at 0 ˚C for 1 h and overnight at room temperature.
Acetone was removed *in vacuo,* the residue was filtered and dried overnight *in vacuo* to give the title compound (155 g) as a light brown solid (y = 63%).
**MS (*m/z*):** 242.2 $[MH]^+$.

**Preparation 2: (1*R*,5S/1*S*,5*R*)-1-[4-(trifluoromethyl)phenyl]-3-azabicyclo[3.1.0]-hexane-2,4-dione**

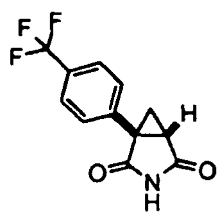

Milled sodium hydroxide (40 g) was added in small portions to a stirred solution of trimethylsulfoxonium iodide (219 g) in DMSO (anhydrous, 2 l). The resulting mixture was allowed to stir at room temperature for 1.5 h. 3-[4-(Trifluoromethyl)phenyl]-1*H*-pyrrole-2,5-dione (120 g) dissolved in DMSO (anhydrous, 0.5 l) was then added dropwise and the resulting mixture was allowed to stir at room temperature for 20 minutes. Temperature was then reduced to 0 ˚C and $NH_4Cl$ (aqueous saturated solution, 2 l) was slowly added, followed by $Et_2O$ (1 l). After separation of the two phases, the aqueous layer was repeatedly extracted with $Et_2O$ (3 x 1 l). Combined organic layers were washed with brine (2 x 1 l) and then dried over $Na_2SO_4$. Evaporation of the solvent gave a light brown solid which was suspended in 1 l of dichloromethane and 1 l of cyclohexane. The mixture was allowed to stir at room temperature for 45 minutes and then filtered to give the title compound (116 g) as white solid (y = 71%).
**MS (*m*/*z*):** 256.1 $[MH]^+$.

**Preparation 3: (1*R*,5*S*/1*S*,5*R*)-1-[4-(trifluoromethyl)phenyl]-3-azabicyclo[3.1.0]-hexane**

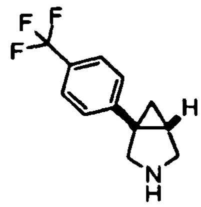

Borane (1M in tetrahydrofuran, 1.4 l) was charged into a 5 l reactor under $N_2$ and cooled at 0 °C. (1*R*,5*S*/1*S*,5*R*)-1-[4-(Trifluoromethyl)phenyl]-3-azabicyclo[3.1.0]hexane-2,4-dione (101 g) dissolved in tetrahydrofuran (anhydrous, 1 l) was then added dropwise with vigorous stirring whereby the temperature was constantly kept below 5 °C and gas evolution was monitored. At the end of the addition the resulting mixture was allowed to stir at 0 °C for 1 h and then at room temperature overnight.
The mixture was then cooled to 0 °C and methanol (200 mL) followed by hydrochloric acid (6 M solution, 0.8 l) were cautiously added monitoring gas evolution. tetrahydrofuran was then removed *in vacuo*, the residue was cooled to 0 °C and sodium hydroxide (5 M solution) was added until pH 9-10 had been reached. The aqueous layer was extracted with $Et_2O$ (3 x 1 l). Removal of solvent *in vacuo* gave the title compound (140 g) as colorless oil.
**MS (*m/z*)**: 228.1 $[MH]^+$.

**Preparation 4: (1*S*,5*R*)-1-[4-(trifluoromethyl)phenyl]-3-azabicyclo[3.1.0]hexane**

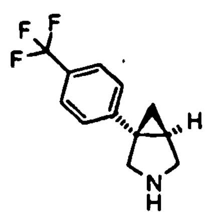

(S) - (+) - Mandelic acid (94 g) was added in portions to a stirred solution of (1*R*,5*S*/1*S*,5*R*)-1-[4-(trifluoromethyl) phenyl]-3-azabicyclo[3.1.0]hexane (140 g) in 1.4 l of tetrahydrofuran. The resulting mixture was stirred at room temperature for 2 h until a white precipitate was formed. The mixture was then warmed up to reflux temperature, stirred for 45 minutes and then slowly cooled down to room temperature. The white solid was collected by filtration and dried *in vacuo.* This material was recrystallised 4 times from tetrahydrofuran (10 volumes) to give 32.5 g of a white solid.
This material was then suspended in sodium hydroxide (1M solution, 400 mL) and $Et_2O$ (400 mL) and allowed to stir at room temperature until complete dissolution. After separation of the two phases, the aqueous layer was extracted again with $Et_2O$ (3 x 250 mL). Combined organic layers were washed with sodium hydroxide (1M solution, 3 x 200 mL) and then dried over $Na_2SO_4$. Evaporation of solvent *in vacuo* gave the title compound (19 g) as white solid (y = 37%).
The absolute configuration of the optical isomers was assigned using comparative VCD (vibrational circular dichroism) and OR (optical rotation) analyses.
The configuration of the title compound was assigned by comparing its experimental VCD spectrum and observed specific rotation to the data observed for (1*S*,5*R*)-1-(3,4-dichlorophenyl)-3-azabicyclo[3.1.0]hexane (see Preparation 11) as the reference sample. The assignment of the absolute configuration of the title compound was confirmed by a single crystal X-ray structure obtained from a crystal of (1*S*,5*R*)-1-[4-(trifluoromethyl)phenyl]-3-azabicyclo[3.1.0]hexane, (S)-(+)-mandelic acid salt. Both, analysis based on the known configuration of the (S)(+)- mandelic acid and on the basis of anomalous dispersion effects confirmed the assignment of the title compound as being (1S,5R)-1-[4-(trifluoromethyl) phenyl]-3-azabicyclo[3,1.0]hexane.
**NMR ($^1H$, $CDCl_3$):** δ 7.51 (d, 2H), 7.25 (d, 2H), 3.20 (d, 1H), 3.0-3.1 (m, 3H), 1.69 (m, 1H), 0.8-1.0 (m, 2H), N<u>H</u> not observed. **MS (*m/z*):** 228.1 $[MH]^+$.

**Analytical chromatography**

Column: chiralcel OD 10 um, 250 x 4.6 mm
Mobile phase: A: n-Hexane; B: Isopropanol +0.1% Isopropyl amine
Gradient: isocratic 2% B

Flow rate: 1 mUmin
UV wavelengh range: 200-400 nm
Analysis time 25 min
**ret. time (min) % a/a**
16.5 0.4 (1*R*,5*S*)-1-[4-(trifluoromethyl)phenyl]-3-azabicyclo[3.1.0]hexane
21.7 99.6 title compound
Specific Optical Rotation: [□]$_D$ = - 10 ˚ ($CDCl_3$, T = 20 ˚C, c ≅ 0.004 g/0.8 mL).

**Preparation 5: (1*S*,5*R*)-3-(3-chloropropyl)-1-[4-(trifluoromethyl)phenyl]-3-azabicyclo[3.1.0]hexane**

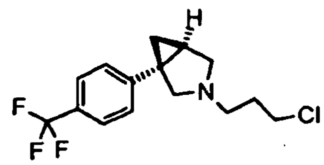

To a solution of (1*S*,5*R*)-1-[4-(trifluoromethyl)phenyl]-3-azabicyclo[3.1.0]hexane (1.00 g) in dry tetrahydrofuran (5 mL), diisopropylethylamine (2.4 mL) and 1-bromo-3-chloropropane (3.7 mL) were added and the resulting mixture was heated at reflux for 3 hours. After cooling at room temperature it was diluted with ethyl acetate (30 mL) washed twice with a saturated solution of $NH_4Cl$ in water (20 mL) and once with a saturated solution of $NaHCO_3$ in water (20 mL), dried over anhydrous $Na_2SO_4$ and concentrated under reduced pressure. The crude product was purified by silica gel chromatography eluting with cyclohexane/EtOAc 7:3 to give the title compound as a colourless oil (1.26 g).
**NMR ($^1$H, $CDCl_3$):** δ 7.50 (d, 2H) 7.19 (d, 2H), 3.59 (t, 2H), 3.33 (d, 1H), 3.09 (d, 1H), 2.58 (m, 2H), 2.66 (dd, 1H), 2.46 (dd, 1H), 1.92 (m, 2H), 1.74 (m, 1H), 1,67 (t, 1H), 0.81 (dd, 1H). **MS (m/z):** 304 $[MH]^+$.

**Preparation 6: methyl bromo(4-methoxyphenyl)acetate**

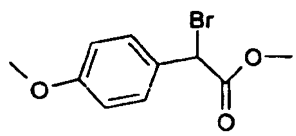

To a mixture of methyl 4-methoxyphenylacetate (20 g, 0.11 mol) and NBS (0.11 mol) in $CCl_4$ (0.2 l) were added 3 drops of 48% HBr and this mixture was heated to reflux for 8 h. The cooled solution was filtered through a pad of silica gel and the filtrate was evaporated *in vacuo* to give 29 g of the title compound as pale yellow oil, which was used in the subsequent step without further purification.
**NMR ($^1$H, $CDCl_3$):** δ 7.3 (d, 2H), 6.8 (d, 2H), 5.1 (s, 1H), 3.8 (s, 3H), 3.5 (s, 3H).

**Preparation 7: dimethyl 1-(4-methoxyphenyl)-1,2-cyclopropanedicarboxylate**

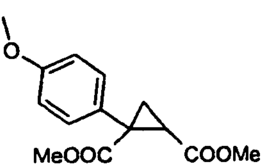

To a stirred slurry of of NaH (4.4 g, 60% in mineral oil) in anhydrous $Et_2O$ (0.3 l )was added methanol (10.3 mL) followed by a solution of methyl bromo(4-methoxyphenyl)acetate (29 g) in methyl acrylate (19.8 mL) (for examples starting from an ethyl phenylacetate derivative ethanol and ethyl acrylate were used, respectively) and methanol (3 mL)

at 0 °C, over a 30 min. The mixture was stirred at 25°C for 24 h and then unreacted NaH was decomposed with 3 mL methanol. Water was added (75 mL), the organic phase separated, dried over $Na_2SO_4$ and filtered. Volatiles were evaporated *in vacuo* to give 31.5 g of the title compound as an oil, which was used in the subsequent step without further purification.

**NMR ($^1$H, $CDCl_3$)**: δ 7.3 (d, 2H), 6.8 (d, 2H), 3.77 (s, 3H), 3.73 (s, 3H), 3.64 (s, 3H), 2.18 (dd, 1H), 2.05 (dd, 1H), 1.46 (dd, 1H). **MS (*m/z*):** 265.4$[MH]^+$.

**Preparation 8: 1-(4-methoxyphenyl)-1,2-cyclopropanedicarboxylic acid**

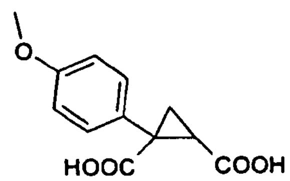

A mixture of dimethyl 1-(4-methoxyphenyl)-1,2-cyclopropanedicarboxylate (31.5 g) and KOH (13.5 g) in 1:1 EtOH:$H_2O$ (240 mL) was heated at reflux for 6 h and then concentrated to half the original volume. The aqueous solution was extracted with $Et_2O$, chilled in ice, and then made acidic with 25 mL of 12N HCl. White crystalline product was collected by filtration and dried under *vacuo* to give 12.8 of the title compound (overall yield from methyl bromo(4-methoxyphenyl)acetate: 50%).

NMR **($^1$H, DMSO)**: δ 12.5 (bs,2H), 7.25 (d, 2H), 6.85 (d, 2H), 3.7 (s, 3H), 2.0 (dd, 1H), 1.85 (dd, 1H), 1.38 (dd, 1H). **MS (*m/z*):** 235.0$[M-H]^-$.

**Preparation 9: (1*R*,5*S*/1*S*,5*R*)-1-[4-(methoxy)phenyl]-3-azabicyclo[3.1.0]hexane-2,4-dione**

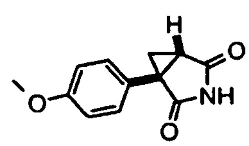

A mixture of 12.8 g of 1-(4-methoxyphenyl)-1,2-cyclopropanedicarboxylic acid and 6.5 g of urea in 300 mL of m-xylene was heated at reflux for 8 h and then concentrated to dryness *in vacuo.* The crude was purified by column chromatography (AcOEt:cyclohexane=1:10 to 4:6) to give 5.5 g of the title compound (y= 46%). **MS (*m/z*)**: 218.1 $[MH]^+$.

**Preparation 10: (1*R*,5*S*/1*S*,5*R*)-1-(4-bromophenyl)-3-azabicyclo[3.1.0]hexane**

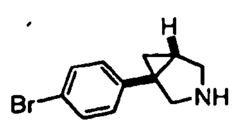

To 20 mL of 1M $BH_3$-tetrahydrofuran, stirred at 0 °C under $N_2$, was slowly added a solution of 1.32 g (5 mmol) of (1*R*,5*S*/1*S*,5*R*)-1-(4-bromophenyl)-3-azabicyclo[3.1.0]hexane-2,4-dione, prepared in analogy to Preparations 6-9, in 20mL of dry tetrahydrofuran. This solution was stirred at room temperature for 15 min and then warmed on a steam bath for 1 h. The solution was then cooled in an ice bath, 2.5 mL of 6 M HCl was added cautiously, and the solvent was removed *in vacuo.* The residual material was combined with 12.5 mL of 5 M NaOH and the mixture was extracted with ether. The ether extract was washed twice with water, dried over $Na_2SO_4$ and filtered to give 1.19 g of the title compound (y= 100%).

**NMR ($^1$H, $CDCl_3$)**: δ 7.35 (d, 2H), 7.02 (d, 2H). 3.25-2.96 (m, 4H). 1.63 (dd, 1H), 1.55 (dd, 1H), 1.30 (dd, 1H), NH not observed. **MS (*m/z*)**: 238.1[MH]$^+$, 1Br.

**Preparation 11: (1*R*,5*S*/1*S*,5*R*)-1-(3,4-dichlorophenyl)-3-azabicyclo[3.1.0]hexane**

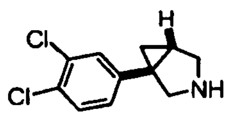

The crude title compound was prepared in 0.36 g yield from commercially available methyl 3,4-dichlorophenylacetate (1 g, 4.57 mmol) following the methods described in preparations 6-10.

The title compound was separated to give the separated enantiomers by preparative chromatography using a chiral column chiralcel AD 10 um, 250 x 21 mm, eluent A: n-hexane; B: isopropanol + 0.1% isopropyl amine, gradient isocratic 2% B, flow rate 7 mL/min, detection UV at 200-400 nm. Retention times given were obtained using an analytical HPLC using a chiral column chiralcel AD 5 um, 250 x 4.6 mm, eluent A: n-hexane; B: isopropanol +0.1% Isopropyl amine, gradient isocratic 2% B, flow rate 1.2 mL/min, detection UV at 200-400 nm.
Enantiomer 1, (1*R*,5*S*)-1-(3,4-dichlorophenyl)-3-azabicyclo[3.1.0]hexane, was recovered in 20 mg yield as white solid from the racemate (60 mg). Rt. = 41 min.
Enantiomer 2, (1*S*,5*R*)-1-(3,4-dichlorophenyl)-3-azabicyclo[3.1.0]hexane, was recovered in 28 mg yield as white solid from the racemate (60 mg). Rt. = 43.4 min.
The absolute configuration of (1*S*,5*R*)-1-(3,4-dichlorophenyl)-3-azabicyclo[3.1.0]hexane was assigned using *ab initio* VCD and *ab initio* OR analyses.
Specific Optical Rotation of (1*S*,5*R*)-1-(3.4-dichlorophenyl)-3-azabicyclo[3.1.0]hexane: $[\square]_D$ = - 67.9 ˚ ($CDCl_3$, T = 20 ˚C, c ≅ 0.01g/mL).
**NMR ($^1$H, $CDCl_3$):** δ 7.35 (d, 1H), 7.27 (s, 1H), 7.02 (dd, 1H), 3.25 (d, 1H), 3.13 (bm, 2H), 3.06 (d, 1H), 1.71 (m, 1H), 0.93 (m, 2H), N<u>H</u> not observed. **MS (*m/z*):** 228 [MH]$^+$,

**Preparation 12: 1-methyl-5-phenyl-1,3-dihydro-2H-imidazole-2-thione**

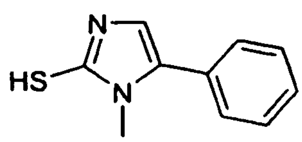

The title compound was prepared as reported in US3505350.

**Preparation 13: 5-phenyl-1,3-thiazole-2(3H)-thione**

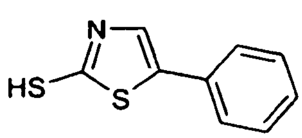

The title compound was prepared as reported in Chem. Ber. 1976, 109, 139-153.

**Preparation 14:** 5-phenyl-1,3,4-thiadiazole-2(3H)-thione

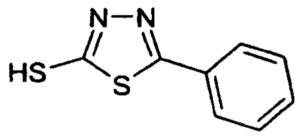

The title compound was prepared as reported in Acta Chemica Scandinavica 1961, 15, 1124-1129.

**Preparation 15: 5-iodo-2-(methylthio)-1,3-thiazole**

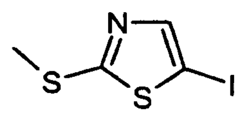

To a solution of 2-(methylthio)-1,3-thiazole (2 g, 15.2 mmol) in DMSO (20 ml), $I_2$ (5.78 g, 22.8 mmol) was added portionwise at 0˚C and the mixture was stirred at room temperature for 4 days. 10% aqueous solution of $Na_2S_2O_5$, (150 ml) was added and the mixture was basified with 10% aqueous $Na_2CO_3$ and extracted three times with diethyl ether. The organic phase was dried ($Na_2SO_4$) and evaporated. The crude was purified by flash chromatography eluting with ethyl acetate-petroleum ether (1-9). The title compound was obtained as a yellow oil (2.4 g).
**MS (*m/z*):** 258 $[M+H]^+$.

**Preparation 16: 3-[2-(methylthio)-1,3-thiazol-5-yl]pyridine**

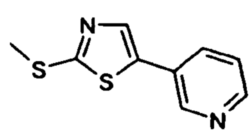

A mixture of 5-iodo-2-(methylthio)-1,3-thiazole (2.1 g, 8.17 mmol), $PPh_3$ (0.221 g, 0.84 mmol), $Pd(OAc)_2$ (0.057 g, 0.25 mmol), $Na_2CO_3$ (1.04 g, 10 mmol) and pyridine-3-boronic acid (1.0 g, 8.17 mmol) in *n*-PrOH (50 ml) was refluxed for 6 hours. Solvent was evaporated and the crude was diluted with water and extracted three times with ethyl acetate. The organic phase was washed with brine, dried ($Na_2SO_4$) and evaporated. The crude product was purified by flash chromatography eluting with dichloromethane-methanol-30%$_{aq}$ammonia (95-5-0.5). The title compound was obtained as a yellow solid (1.4 g, 82% yield). **MS (*m/z*)**: 209,1 $[M+H]^+$.

**Preparation 17: 3-[2-(methylsulfinyl)-1,3-thiazol-5-yl]pyridine**

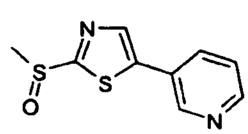

A solution of 3-[2-(methylthio)-l.3-thiazol-5-yl]pyridine (1.4 g, 6.73 mmol) in dichloromethane (140 ml) was cooled to 0˚C and a solution of MCPBA (77%, 1.51 g. 6.73 mmol) in dichloromethane (30 ml) was added dropwise during 1 hour. The mixture was stirred at the same temperature for 30 minutes, then washed with aqueous saturated $NaHCO_3$, brine, dried ($Na_2SO_4$) and evaporated. The crude was triturated with *i*-$Pr_2O$. The title compound was obtained as yellow solid (1.2 g, 79% yield). **MS (*m/z*)**: 225,1 $[M+H]^+$.

**Preparation 18: 5-(3-pyridinyl)-1,3-thiazole-2(3H)-thione**

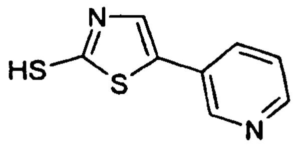

To a solution of 3-[2-(methylsulfinyl)-1,3-thiazol-5-yl]pyridine (1.2 g) in dichloromethane (20 ml), TFAA (12 ml) was added and the mixture was stirred at reflux for 6 hours. The solvent was evaporated and the residue was purified by flash chromatography with dichloromethane-methanol-30%$_{aq}$ammonia (90-10-0.5). A dark brown solid (0.515 g) was obtained and it was sequentially triturated with *i*-PrOH, EtOH and MeOH. The title compound was obtained as a light brown solid (0.34 g) (m.p. 299-300°C). **MS (*m/z*):** 194,9 $[M+H]^+$.

**Preparation 19: 1-methyl-2-(methylthio)-1H-imidazole**

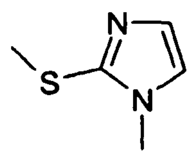

The title compound was prepared as reported in J. Heteroc. Chem. 1995, 227-234.

**Preparation 20: 5-iodo-1-methyl-2-(methylthio)-1*H*-imidazole**

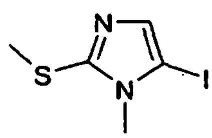

The title compound was obtained as a yellow powder (2.8 g) starting from 1-methyl-2-(methylthio)-1H-imidazole (12.8 g) following the same procedure described for the synthesis of 5-iodo-2-(methylthio)-1,3-thiazole. **MS (*m/z*):** 254,8 $[M+H]^+$.

**Preparation 21: 3-[1-methyl-2-(methylthio)-1H-imidazol-5-yl]pyridine**

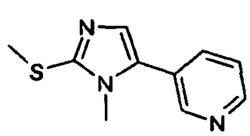

The title compound was obtained as a yellow oil (0.8 g) starting from 5-iodo-1-methyl-2-(methylthio)-1H-imidazole (2.8 g) following the same procedure described for the synthesis of 3-[2-(methylthio)-1,3-thiazol-5-yl]pyridine.

**MS (*m/z*)**: 206,0 $[M+H]^+$.

**Preparation 22: 3 3-[1-methyl-2-(methylsulfinyl)-1H-imidazol-5-yl]pyridine**

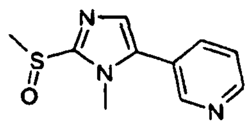

The title compound was obtained as a yellow oil (0.55 g) starting from 3-[1-methyl-2-(methylthio)-1H-imidazol-5-yl]pyridine (0.8 g) following the same procedure described for the synthesis of 3-[2-(methylsulfinyl)-1,3-thiazol-5-yl] pyridine. **MS (*m/z*):** 222,0 $[M+H]^+$.

**Preparation 23: 1-methyl-5-(3-pyridinyl)-1,3-dihydro-2H-imidazole-2-thione**

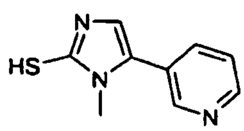

The title compound was obtained as a pale yellow solid (0.117 g) starting from 3-[1-methyl-2-(methylsulfinyl)-1H-imidazol-5-yl]pyridine (0.5 g) following the same procedure described for the synthesis of 5-(3-pyridinyl)-1,3-thiazole-2(3H)-thione. **MS (*m/z*):** 192,2 $[M+H]^+$.

**Examples 1-16 (Table 1):**

To a solution of thioaryl (0.082 mmol) in dry acetonitrile (2 ml) 2-*tert*-butylimino-2-diethylamino-1,3-dimethyl-perhydro-1,3,2-diaza-phosphorine on polystyrene (56 mg, 2.2 mmol/g) was added and the resulting mixture was shaken for 30 minutes at room temperature then (1*S*,5*R*)-3-(3-chloropropyl)-1-[4-(trifluoromethyl)phenyl]-3-azabicyclo[3.1.0] hexane (25 mg) was added and the resulting mixture was shaken at 50° C overnight. After cooling the resin was filtered off, washed with methanol (2ml) and then the solvent was removed under reduced pressure. Purifications were carried out using mass directed HPLC using a Waters XTerra Prep MS C18 10μm, 100 x 19 mm column using the following conditions

Mobile phase: A: NH4HCO3 sol. 10 mM, pH10; B: CH3CN
Gradient 1: 30% (B) for 1 min, from 30% (B) to 95% (B) in 9 min, 95% (B) for 3 min
Flow rate: 17 ml/min
UV wavelength range: 210-350 nm
Mass range: 100-900 amu
Ionization: ES+

Then solvent was removed under reduced pressure to give title compounds as free bases.

HPLC:

Analytical

Column: X Terra MS C18 5 μm, 50 x 4.6 mm
Mobile phase: A: NH4HCO3 sol. 10 mM, pH10; B: CH3CN
Gradient: 30% (B) for 1 min, from 30% (B) to 95% (B) in 9 min, 95% (B) for 3 min
Flow rate: 1 ml/min
UV wavelength range: 210-350 nm
Mass range: 100-900 amu

Ionization: ES+

The thioaryls, not herein described, used in the preparations may be commercially available or may be prepared according to procedures described in the literature (see for example: Kjellin, Gunnar; Sandstrom, Jan. Chem; Center, Univ. Lund, Lund, Swed; Acta Chemica Scandinavica (1947-1973) (1969), 23(8), 2879-87; European Patent Application EP263066).

**Table 1**

| Ex. N. | *Structure* | *Compound Name* | *Retention time (min)* | *MS* (*m*/*z*) *[MH]+* |
|---|---|---|---|---|
| **1** | 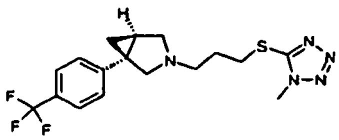 | (1S,5R)-3-{3-[(1-methyl-1H-tetrazol-5-yl)thio]propyl}-1-[4-(trifluoromethyl) phenyl]-3-azabicyclo [3.1.0]hexane | **7.74** | **384** |
| **2** | 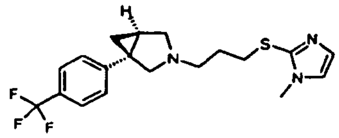 | (1S,5R)-3-{3-[(1-methyl-1H-imidazol-2-yl)thio]propyl}-1-[4-(trifluoromethyl) phenyl]-3-azabicyclo [3.1.0]hexane | **7.52** | **382** |
| **3** | 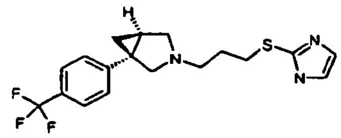 | (1*S*,5*R*)-3-[3-(1H-imidazol-2-ylthio) propyl]-1-[4-(trifluoromethyl) phenyl]-3-azabicyclo [3.1.0]hexane | **6.57** | **368** |
| **4** | 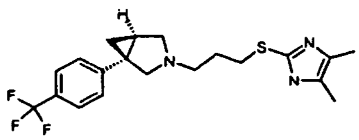 | (1S,5R)-3-{3-[(4,5-dimethyl-1H-imidazol-2-yl)thio] propyl}-1-[4-(trifluoromethyl) phenyl]-3-azabicyclo [3.1.0]hexane | **7.14** | **396** |
| **5** | 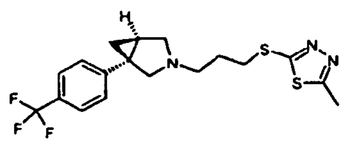 | (1*S*,5*R*)-3-{3-[(5-methyl-1,3,4-thiadiazol-2-yl)thio] propyl}-1-[4-(trifluoromethyl) phenyl]-3-azabicyclo [3.1.0]hexane | **8.49** | **401** |
| **6** | 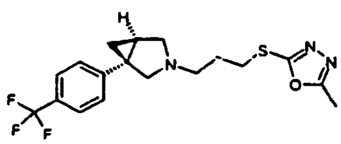 | (1S,5R)-3-{3-[(5-methyl-1,3,4-oxadiazol-2-yl)thio] propyl}-1-[4-(trifluoromethyl) phenyl]-3-azabicyclo [3.1.0]hexane | **7.95** | **384** |
| **7** | 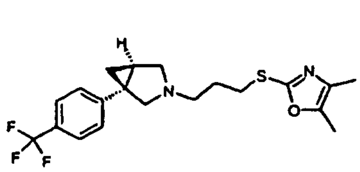 | (1S,5R)-3-{3-[(4,5-dimethyl-1,3-oxazol-2-yl)thio]propyl}-1-[4-(trifluoromethyl) phenyl]-3-azabicyclo [3.1.0]hexane | **9.84** | **397** |

(continued)

| Ex. N. | *Structure* | *Compound Name* | *Retention time (min)* | *MS (m/z) [MH]+* |
|---|---|---|---|---|
| **8** | 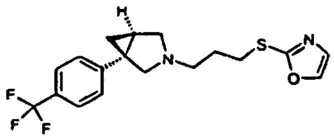 | (1S,5R)-3-[3-(1,3-oxazol-2-ylthio) propyl]-1-[4-(trifluoromethyl) phenyl]-3-azabicyclo [3.1.0]hexane | **8.84** | **369** |
| **9** | 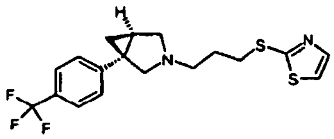 | (1S,5R)-3-[3-(1,3-thiazol-2-ylthio) propyl]-1-[4-(trifluoromethyl) phenyl]-3-azabicyclo [3.1.0]hexane | **9.46** | **385** |
| **10** | 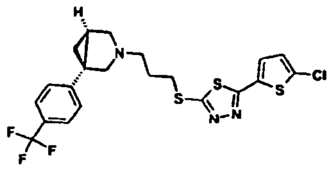 | (1S,5R)-3-(3-{[5-(5-chloro-2-thienyl)-1,3,4-thiadiazol-2-yl] thio}propyl)-1-[4-(trifluoromethyl) phenyl]-3-azabicyclo [3.1.0]hexane | **5.92** | **501** |
| **11** | 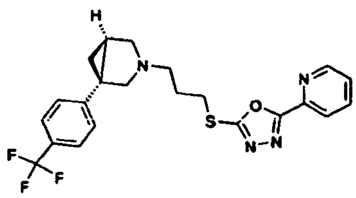 | (1S,5R)-3-(3-{[5-(2-pyridinyl)-1,3,4-oxadiazol-2-yl]thio} propyl)-1-[4-(trifluoromethyl) phenyl]-3-azabicyclo [3.1.0]hexane | **4.19** | **446** |
| **12** | 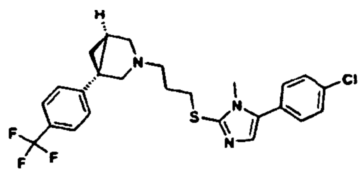 | (1S,5R)-3-(3-{[5-(4-chlorophenyl)-1-methyl-1H-imidazol-2-yl]thio}propyl)-1-[4-(trifluoromethyl) phenyl]-3-azabicyclo [3.1.0]hexane | **5.10** | **491** |
| **13** | 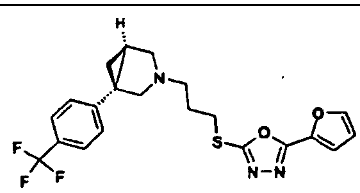 | (1S,5R)-3-(3-{[5-(2-furanyl)-1,3,4-oxadiazol-2-yl]thio} propyl)-1-[4-(trifluoromethyl) phenyl]-3-azabicyclo [3.1.0]hexane | **4.61** | **435** |
| **14** | 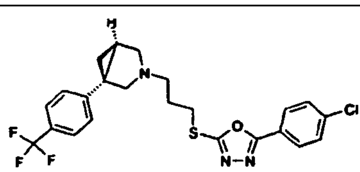 | (1S,5R)-3-(3-{[5-(4-chlorophenyl)-1,3,4-oxadiazol-2-yl]thio} propyl)-1-[4-(trifluoromethyl) phenyl]-3-azabicyclo [3.1.0]hexane | **5.54** | **479** |

(continued)

| Ex. N. | Structure | Compound Name | Retention time (min) | MS (m/z) [MH]+ |
|---|---|---|---|---|
| **15** | 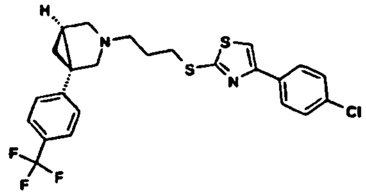 | (1S,5R)-3-(3-{[4-(4-chlorophenyl)-1,3-thiazol-2-yl]thio} propyl)-1-[4-(trifluoromethyl) phenyl]-3-azabicyclo [3.1.0]hexane | **6.28** | **494** |
| **16** | 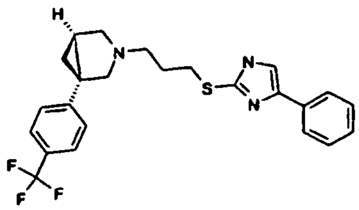 | (1S,5R)-3-{3-[(4-phenyl-1H-imidazol-2-yl)thio]propyl}-1-[4-(trifluoromethyl) phenyl]-3-azabicyclo [3.1.0]hexane | **4.12** | **443** |

**Example 17: (1*S*,5*R*)-3-(3-{[5-(3-pyridinyl)-1,3-thiazol-2-yl]thio}propyl)-1-[4-(trifluoromethyl)phenyl]-3-azabicyclo[3.1.0]hexane hydrochloride**

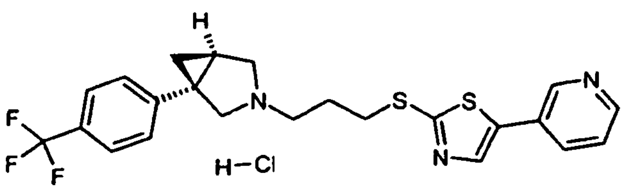

The title compound was prepared in analogy to the method described in Example 1 in 19 mg yield as a white slightly hygroscopic solid starting from (1S,5R)-3-(3-chloropropyl)-1-[4-(trifluoromethyl)phenyl]-3-azabicyclo[3.1.0]hexane (50 mg) and 5-(3-pyridinyl)-1,3-thiazole-2(3H)-thione (38 mg).
**1H NMR (500 MHz, DMSO-$d_6$)** ppm 10.57 (br. s., 1 H) 8.88 - 8.93 (m, 1 H) 8.55 - 8.59 (m, 1 H) 8.29 - 8.30 (m, 1 H) 8.11 - 8.11 (m, 1 H) 7.69 (d, 2 H) 7.52 - 7.57 (m, 1 H) 7.48 (d, 2 H) 4.06 (dd, 1 H) 3.73 (dd, 1 H) 3.48 - 3.66 (m, 2 H) 3.29 - 3.39 (m, 4 H) 2.25 - 2.31 (m, 1 H) 2.17 - 2.25 (m, 2 H) 1.68 - 1.74 (m, 1 H) 1.15 - 1.20 (m, 1 H). **MS (m/z)**: 462[MH]$^+$.

**Example 18: (1*S*,5*R*)-3-(3-{[1-methyl-5-(3-pyridinyl)-1*H*-imidazol-2-yl]thio}propyl)-1-[4-(trifluoromethyl)phenyl]-3-azabicyclo[3.1.0]hexane hydrochloride**

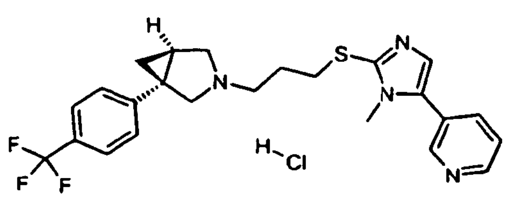

The title compound was prepared in analogy to the method described in Example 1 in 18 mg yield as a white slightly hygroscopic solid starting from (1S,5R)-3-(3-chloropropyl)-1-[4-(trifluoromethyl)phenyl]-3-azabicyclo[3.1.0]hexane (50 mg) and 1-methyl-5-(3-pyridinyl)-1,3-dihydro-2H-imidazole-2-thione (35 mg). **MS (m/z):** 459 [MH]$^+$.

**Example 19: (1S,5R)-3-{3-[(1-methyl-5-phenyl-1H-imidazol-2-yl)thio]propyl}-1-[4-(trifluoromethyl)phenyl]-3-azabicyclo[3.1.0]hexane hydrochloride**

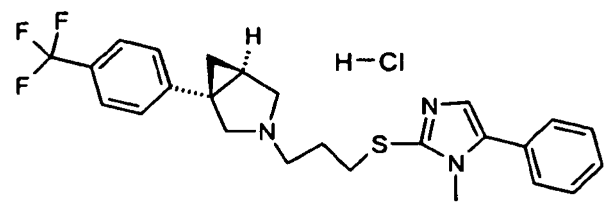

The title compound was prepared in analogy to the method described in Example 1 in 20 mg yield as a white slightly hygroscopic solid starting from (1S,5R)-3-(3-chloropropyl)-1-[4-(trifluoromethyl)phenyl]-3-azabicyclo[3.1.0]hexane (40 mg) and 1-methyl-5-phenyl-1,3-dihydro-2H-imidazole-2-thione (25 mg).
**NMR (1H, DMSO-$d_6$)** ppm 1.18 - 1.26 (m, 1 H) 1.60 - 1.78 (m, 1 H) 2.00 - 2.18 (m, 2 H) 2.25 - 2.34 (m, 1 H) 3.08 - 3.79 (m, 7 H) 3.66 (s, 3 H) 4.04 (d, 1 H) 7.39 - 7.58 (m, 7 H) 7.70 (d, 2 H) 10.59 (br. s., 1 H)

**Example 20: (1*S*,5*R*)-3-{3-[(5-phenyl-1,3-thiazol-2-yl)thio]propyl}-1-[4-(trifiuoromethyl)phenyl]-3-azabicyclo [3.1.0]hexane hydrochloride**

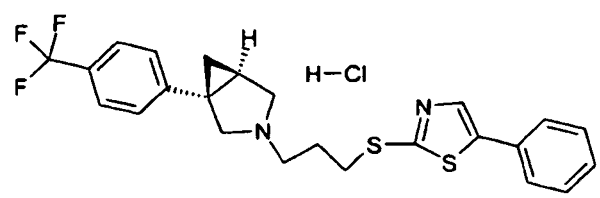

The title compound was prepared in analogy to the method described in Example 1 in 58 mg yield as a white slightly hygroscopic solid starting from (1S,5R)-3-(3-chloropropyl)-1-[4-(trifluoromethyl)phenyl]-3-azabicyclo[3.1.0]hexane (40 mg) and 5-phenyl-1,3-thiazole-2(3H)-thione (31 mg).
**NMR (1H, DMSO-$d_6$)** ppm 1.10 - 1.30 (m, 1 H) 1.50 - 1.71 (m, 1 H) 2.12 - 2.24 (m, 2 H) 2.24 - 2.32 (m, 1 H) 3.25 - 3.45 (m, 4 H) 3.44 - 3.56 (m, 1 H) 3.58 - 3.68 (m, 1 H) 3.67 - 3.79 (m, 1 H) 3.95 - 4.14 (m, 1 H) 7.34 (t, 1 H) 7.43 (t, 2 H) 7.49 (d, 2 H) 7.61 (d, 2 H) 7.69 (d, 2 H) 8.12 - 8.16 (m, 1 H) 10.32 (br. s., 1 H)

**Example 21: (1*S*,5*R*)-3-{3-[(5-phenyl-1,3,4-thladiazol-2-yl)thlo]propyl}-1-[4-(trifluoromethyl)phenyl]-3-azabicyclo[3.1.0]hexane hydrochloride**

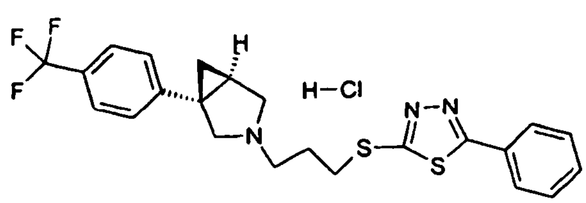

The title compound was prepared in analogy to the method described in Example 1 in 45 mg yield as a white slightly hygroscopic solid starting from (1S,5R)-3-(3-chloropropyl)-1-[4-(trifluoromethyl)phenyl]-3-azabicyclo[3.1.0]hexane (40 mg) and 5-phenyl-1,3,4-thiadiazole-2(3H)-thione (31 mg).
**NMR (1H, DMSO-$d_6$)** ppm 1.00 - 1.28 (m, 1 H) 1.56 - 1.76 (m, 1 H) 2.19 - 2.33 (m, 3 H) 3.23 - 3.42 (m, 2 H) 3.43 (t, 2

H) 3.48 - 3.56 (m, 1 H) 3.62 (dd, 1 H) 3.69 - 3.80 (m, 1 H) 4.01 - 4.15 (m, 1 H) 7.48 (d, 2 H) 7.53 - 7.59 (m, 3 H) 7.69 (d, 2 H) 7.90 (d, 2 H) 10.40 (br. s., 1 H)

It is to be understood that the present invention covers all combinations of particular groups described herein above.

The application of which this description and claims forms part may be used as a basis for priority in respect of any subsequent application. The claims of such subsequent application may be directed to any feature or combination of features described herein. They may take the form of product, composition, process, or use claims and may include, by way of example and without limitation, the following claims.

## Claims

**1.** A compound of formula (I) or a salt thereof:

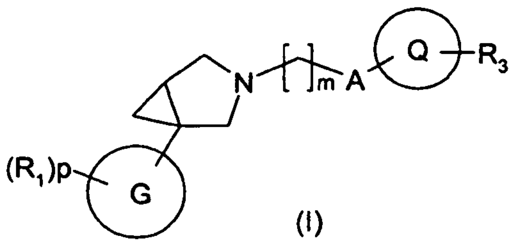

(I)

wherein

- G is selected from a group consisting of: phenyl, pyridyl, benzothiazolyl and indazolyl;
- p is 0, 1, 2, 3, 4 or 5;
- $R_1$ is independently selected from a group consisting of: halogen, hydroxy, cyano, $C_{1-4}$alkyl, halo$C_{1-4}$alkyl, $C_{1-4}$alkoxy, halo$C_{1-4}$alkoxy, $C_{1-4}$alkanoyl and $SF_5$; or corresponds to a group $R_2$;
- $R_2$ is selected from a group consisting of: isoxazolyl, $-CH_2$-N-pyrrolyl, 1,1-dioxido-2-isothiazolidinyl, thienyl, thiazolyl, pyridyl and 2-pyrrolidinonyl, and such a group is optionally substituted by one or two substituents selected from a group consisting of: halogen, cyano, $C_{1-4}$alkyl, halo$C_{1-4}$alkyl, $C_{1-4}$alkoxy and $C_{1-4}$alkanoyl;
- m is 2, 3, 4 or 5;
- A is S, O or $-CH_2-$;
- Q is a 5-membered heteroaromatic group other than triazolyl, which 5-membered heteroaromatic group is optionally substituted by one or two $C_{1-4}$alkyl; and
- $R_3$ is hydrogen, $C_{1-4}$alkyl, phenyl, a heterocyclyl group, a 5- or 6-membered heteroaromatic group or a 8- to 11-membered bicyclic group, any of which groups is optionally substituted by 1, 2, 3 or 4 substituents selected from the group consisting of: halogen, cyano, $C_{1-4}$alkyl, halo$C_{1-4}$alkyl, $C_{1-4}$alkoxy, $C_{1-4}$alkanoyl and $SF_5$;

and when $R_1$ corresponds to $R_2$, p is 1.

**2.** A compound as claimed in claim 1, wherein G is phenyl.

**3.** A compound as claimed in claim 1, wherein $R_1$ is trifluoromethyl.

**4.** A compound as claimed in any of claims 1-3, wherein A is sulfur.

**5.** A compound as claimed in any of claims 1-4, wherein m is 3.

**6.** A compound as claimed in claim 1, having a general formula (IC) or a salt thereof:

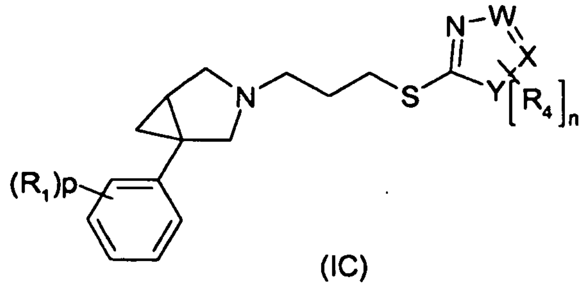

(IC)

wherein $R_1$ and p are as defined for formula (I), n is 0, 1 or 2, $R_4$ is hydrogen or $C_{1-4}$alkyl, W is C or N, X is N or $CR_3$ and Y is O, S, NH or $NC_{1-4}$alkyl, wherein $R_3$ is hydrogen, $C_{1-4}$alkyl, phenyl, a heterocyclyl group, a 5- or 6-membered heteroaromatic group or a 8- to 11-membered bicyclic group, any of which groups is optionally substituted by 1, 2, 3 or 4 substituents selected from the group consisting of: halogen, cyano, $C_{1-4}$alkyl, halo$C_{1-4}$alkyl, $C_{1-4}$alkoxy, $C_{1-4}$alkanoyl and $SF_5$.

**7.** A compound as claimed in any of claims 1-6, wherein Q is imidazolyl, thiadiazolyl, oxadiazolyl, thiazolyl, oxazolyl, tetrazolyl or thienyl, each of which is optionally substituted by a $C_{1-4}$alkyl group.

**8.** A compound as claimed in claim 1, having a formula (ID) or a salt thereof:

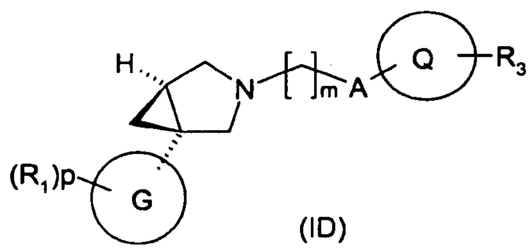

(ID)

wherein G, p, $R_1$, m, A, Q and $R_3$ are defined in claim 1.

**9.** A compound as claimed in claim 1, having a general formula (IG) or a salt thereof:

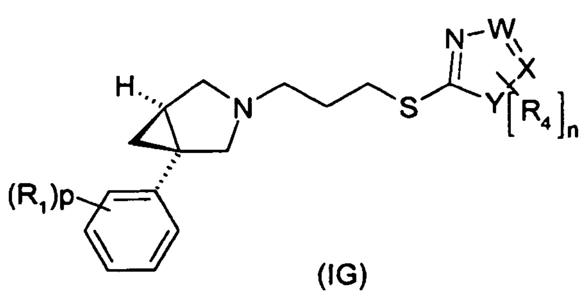

(IG)

wherein $R_1$ and p are as defined for formula (I), n is 0, 1 or 2, $R_4$ is hydrogen or $C_{1-4}$alkyl, W is C or N, X is N or $CR_3$ and Y is O, S, NH or $NC_{1-4}$alkyl, wherein $R_3$ is hydrogen, $C_{1-4}$alkyl, phenyl, a heterocyclyl group, a 5- or 6-membered heteroaromatic group or a 8- to 11-membered bicyclic group, any of which groups is optionally substituted by 1, 2, 3 or 4 substituents selected from the group consisting of: halogen, cyano, $C_{1-4}$alkyl, halo$C_{1-4}$alkyl, $C_{1-4}$alkoxy, $C_{1-4}$alkanoyl and $SF_5$.

**10.** A compound as claimed in claim 1, which is

(1S,SR)-3-{3-[(1-methyl-1H-tetrazol-5-yl)thio]propyl}-1-[4-(trifluoromethyl)phenyl]-3-azabicyclo[3.1.0]hexane;
(1S,5R)-3-{3-[(1-methyl-1H-imidazol-2-yl)thio]propyl}-1-[4-(trifluoromethyl)phenyl]-3-azabicyclo[3.1.0]hexane;
(1S,5R)-3-[3-(1H-imidazol-2-ylthio)propyl]-1-[4-(trifluoromethyl)phenyl]-3-azabicyclo[3.1.0]hexane;
(1S,5R)-3-{3-[(4,5-dimethyl-1H-imidazol-2-yl)thio]propyl}-1-[4-(trifluoromethyl)phenyl]-3-azabicyclo[3.1.0]hexane;
(1S,5R)-3-{3-[(5-methyl-1,3,4-thiadiazol-2-yl)thio]propyl}-1-[4-(trifluoromethyl)phenyl]-3-azabicyclo[3.1.0]hexane;
(1S,5R)-3-{3-[(5-methyl-1,3,4-oxadiazol-2-yl)thio]propyl}-1-[4-(trifluoromethyl)phenyl]-3-azabicyclo[3.1.0]hexane;
(1S,5R)-3-{3-[(4,5-dimethyl-1,3-oxazol-2-yl)thio]propyl}-1-[4-(trifluoromethyl)phenyl]-3-azabicyclo[3.1.0]hexane;
(1S,5R)-3-[3-(1,3-oxazol-2-ylthio)propyl]-1-[4-(trifluoromethyl)phenyl]-3-azabicydo[3.1.0]hexane;
(1S,5R)-3-[3-(1,3-thiazol-2-ylthio)propyl]-1-[4-(trifluoromethyl)phenyl]-3-azabicyclo[3.1.0]hexane;
(1S,5R)- 3-(3-{[5-(3-pyridinyl)-1,3-thiazol-2-yl]thio}propyl)-1-[4-(trifluoromethyl)phenyl]-3-azabicyclo[3.1.0]hexane;
(1S,5R)-3-(3-{[1-methyl-5-(3-pyridinyl)-1H-imidazol-2-yl]thio}propyl)-1-[4-(trifluoromethyl)phenyl]-3-azabicyclo[3.1.0]hexane;
(1S, 5R)- 3- {3-[(1- methyl- 5- phenyl- 1H- imidazol- 2- yl) thio] propyl}- 1-[4-(trifluoromethyl) phenyl]- 3- azabicyclo[3.1.0]hexane;
(1S,5R)-3-{3-[(5-phenyl-1,3-thiazol-2-yl)thio]propyl}-1-[4-(trifluoromethyl)phenyl]-3-azabicyclo[3.1.0]hexane;
(1S,5R)-3-{3-[(5-phenyl-1,3,4-thiadiazol-2-yl)thio]propyl}-1-[4-(trifluoromethyl)phenyl]-3-azabicyclo[3.1.0]hexane;
(1S,5R)-3-(3-{[5-(5-chloro-2-thienyl)-1,3,4-thiadiazol-2-yl]thio}propyl)-1-[4-(trifluoromethyl)phenyl]-3-azabicyclo[3.1.0]hexane;
(1S,5R)-3-(3-{[5-(2-pyridinyl)-1,3,4-oxadiazol-2-yl]thio}propyl)-1-[4-(trifluoromethyl)phenyl]-3-azabicydo[3.1.0]hexane;
(1S,5R)-3-(3-[5-(4-chlorophenyl)-1-methyl-1H-imidazol-2-yl]thiolpropyl)-1-[4-(trifluoromethyl)phenyl]-3-azabicyclo[3.1.0]hexane;
(1S,5R)-3-(3-{[5-(2-furanyl)-1,3,4-oxadiazol-2-yl]thio}propyl)-1-[4-(trifluoromethyl)phenyl]-3-azabicyclo[3.1.0]hexane;
(1S,5R)-3-(3-{[5-(4-chlorophenyl)-1,3,4-oxadiazol-2-yl]thio}propyl)-1-[4-(trifluoromethyl)phenyl]-3-azabicyclo[3.1.0]hexane;
(1S,5R)-3-(3-{[4-(4-chlorophenyl)-1,3-thiazol-2-yl]thio}propyl)-1-[4-(trifluoromethyl)phenyl]-3-azabicyclo[3.1.0]hexane;
(1S,5R)-3-{3-[(4-phenyl-1H-imidazol-2-yl)thio]propyl}-1-[4-(trifluoromethyl)phenyl]-3-azabicyclo[3.1.0]hexane;

or a salt thereof.

**11.** A process for preparing a compound as defined in claim 1, the process comprising, for a compound of formula (I) wherein A is S, reacting a compound of formula (V):

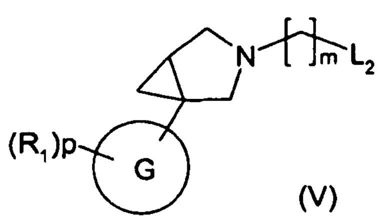

(V)

wherein $R_1$, p, G and m are as defined in claim 1 and $L_2$ is a leaving group, with a compound of formula (VI):

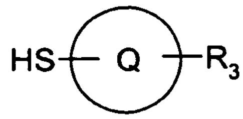

(VI)

wherein Q and $R_3$ are as defined in claim 1;
and thereafter optionally

(i) removing any protecting group(s); and/or
(ii) forming a salt; and/or
(iii) converting a compound as claimed in claim 1 to another compound as claimed in claim 1.

**12.** Use of a compound as claimed in any of claims 1-10 in the manufacture of a medicament for the treatment psychosis or a psychotic condition, substance abuse, premature ejaculation or cognition impairment in a mammal.

**13.** Use as claimed in claim 12, wherein the condition is substance abuse.

**14.** Use as claimed in claim 12, wherein the psychotic condition is schizophrenia.

**15.** A compound as claimed in any of claims 1-10 for use in therapy.

**16.** A compound as claimed in any of claims 1-10 for use in the treatment of psychosis or a psychotic condition, of substance abuse, premature ejaculation or cognition impairment.

**17.** A compound as claimed in any of claims 1-10 for use in the treatment of schizophrenia.

**18.** A compound as claimed in any of claims 1-10 for use in the treatment of substance abuse.

**19.** A pharmaceutical composition comprising a compound as claimed in any of claims 1-10 and a pharmaceutically acceptable carrier.

**Patentansprüche**

**1.** Eine Verbindung der Formel (I) oder ein Salz davon:

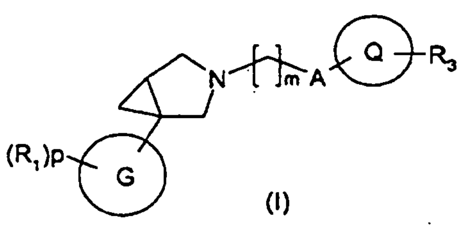

(I)

wobei

- G ausgewählt ist aus einer Gruppe bestehend aus: Phenyl, Pyridyl, Benzothiazolyl und Indazolyl;
- p gleich 0,1, 2, 3, 4 oder 5 ist;
- $R_1$ unabhängig ausgewählt ist aus einer Gruppe bestehend aus: Halogen, Hydroxy, Cyano, $C_{1-4}$-Alkyl, Halogen-$C_{1-4}$-alkyl, $C_{1-4}$-Alkoxy, Halogen-$C_{1-4}$-alkoxy, $C_{1-4}$-Alkanoyl und $SF_5$; oder einem Rest $R_2$ entspricht;
- $R_2$ ausgewählt ist aus einer Gruppe bestehend aus: Isoxazolyl, -$CH_2$-N-Pyrrolyl, 1,1-Dioxido-2-isothiazolidinyl, Thienyl, Thiazolyl, Pyridyl und 2-Pyrrolidinonyl, und ein derartiger Rest gegebenenfalls mit einem oder zwei Substituenten, ausgewählt aus einer Gruppe bestehend aus: Halogen, Cyano, $C_{1-4}$-Alkyl, Halogen-$C_{1-4}$-alkyl,

$C_{1-4}$-Alkoxy und $C_{1-4}$-Alkanoyl, substituiert ist;
• m gleich 2, 3, 4 oder 5 ist;
• A gleich S, O oder $-CH_2-$ ist;
• Q ein 5-gliedriger heteroaromatischer, von Triazolyl verschiedener Rest ist, wobei der 5-gliedrige heteroaromatische Rest gegebenenfalls mit einem oder zwei $C_{1-4}$-Alkylresten substituiert ist; und
• $R_3$ Wasserstoff, $C_{1-4}$-Alkyl, Phenyl, ein Heterocyclylrest, ein 5- oder 6-gliedriger heteroaromatischer Rest oder ein 8- bis 11-gliedriger bicyclischer Rest ist, wobei jeder der Reste gegebenenfalls mit 1, 2, 3 oder 4 Substituenten, ausgewählt aus der Gruppe bestehend aus: Halogen, Cyano, $C_{1-4}$-Alkyl, Halogen-$C_{1-4}$-alkyl, $C_{1-4}$-Alkoxy, $C_{1-4}$-Alkanoyl und $SF_5$, substituiert ist;

und wenn $R_1$ $R_2$ entspricht, p 1 ist.

2. Eine Verbindung wie in Anspruch 1 beansprucht, wobei G Phenyl ist.

3. Eine Verbindung wie in Anspruch 1 beansprucht, wobei $R_1$ Trifluormethyl ist.

4. Eine Verbindung wie in einem der Ansprüche 1 bis 3 beansprucht, wobei A Schwefel ist.

5. Eine Verbindung wie in einem der Ansprüche 1 bis 4 beansprucht, wobei m 3 ist.

6. Eine Verbindung wie in Anspruch 1 beansprucht mit einer allgemeinen Formel (IC) oder ein Salz davon:

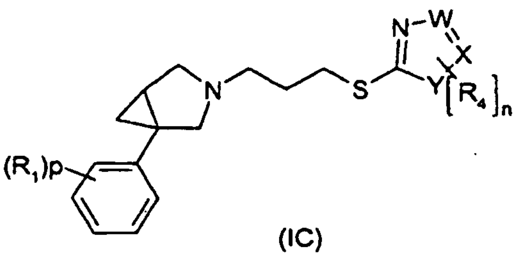

(IC)

wobei $R_1$ und p wie für Formel (I) definiert sind, n gleich 0, 1 oder 2 ist, $R_4$ Wasserstoff oder $C_{1-4}$-Alkyl ist, W gleich C oder N ist, X gleich N oder $CR_3$ ist und Y gleich O, S, NH oder $NC_{1-4}$-Alkyl ist, wobei $R_3$ Wasserstoff, $C_{1-4}$-Alkyl, Phenyl, ein Heterocyclylrest, ein 5- oder 6-gliedriger heteroaromatischer Rest oder ein 8- bis 11-gliedriger bicyclischer Rest ist, wobei jeder der Reste gegebenenfalls mit 1, 2, 3 oder 4 Substituenten, ausgewählt aus der Gruppe bestehend aus: Halogen, Cyano, $C_{1-4}$-Alkyl, Halogen-$C_{1-4}$-alkyl, $C_{1-4}$-Alkoxy, $C_{1-4}$-Alkanoyl und $SF_5$, substituiert ist.

7. Eine Verbindung wie in einem der Ansprüche 1 bis 6 beansprucht, wobei Q Imidazolyl, Thiadiazolyl, Oxadiazolyl, Thiazolyl, Oxazolyl, Tetrazolyl oder Thienyl ist, von denen jeder gegebenenfalls mit einem $C_{1-4}$-Alkylrest substituiert ist.

8. Eine Verbindung wie in Anspruch 1 beansprucht mit einer Formel (ID) oder ein Salz davon:

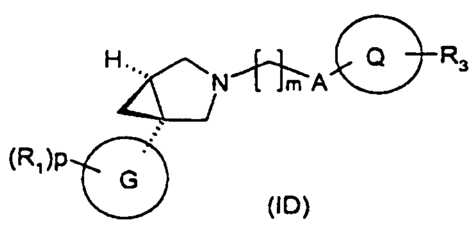

(ID)

wobei G, p, $R_1$, m, A, Q und $R_3$ in Anspruch 1 definiert sind.

9. Eine Verbindung wie in Anspruch 1 beansprucht mit einer allgemeinen Formel (IG) oder ein Salz davon:

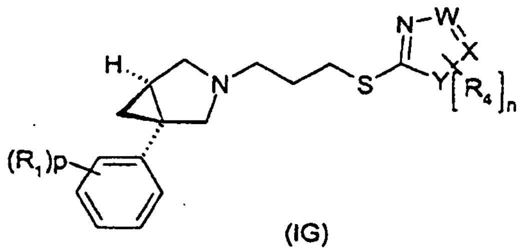

(IG)

wobei $R_1$ und p wie für Formel (I) definiert sind, n gleich 0, 1 oder 2 ist, $R_4$ Wasserstoff oder $C_{1-4}$-Alkyl ist, W gleich C oder N ist, X gleich N oder $CR_3$ ist und Y gleich O, S, NH oder $NC_{1-4}$-Alkyl ist, wobei $R_3$ Wasserstoff, $C_{1-4}$-Alkyl, Phenyl, ein Heterocyclylrest, ein 5- oder 6-gliedriger heteroaromatischer Rest oder ein 8- bis 11-gliedriger bicyclischer Rest ist, wobei jeder der Reste gegebenenfalls mit 1, 2, 3 oder 4 Substiuenten, ausgewählt aus der Gruppe bestehend aus: Halogen, Cyano, $C_{1-4}$-Alkyl, Halogen-$C_{1-4}$-alkyl, $C_{1-4}$-Alkoxy, $C_{1-4}$-Alkanoyl und $SF_5$, substituiert ist.

**10.** Eine Verbindung wie in Anspruch 1 beansprucht, nämlich
(1S,5R)-3-{3-[(1-Methyl-1H-tetrazol-5-yl)thio]propyl}-1-[4-(trifluormethyl)phenyl]-3-azabicyclo[3.1.0]hexan;
(1S,5R)-3-{3-[(1-Methyl-1H-imidazol-2-yl)thio]propyl}-1-[4-(trifluormethyl)phenyl]-3-azabicyclo[3.1.0]hexan;
(1S,5R)-3-[3-(1H-Imidazol-2-ylthio)propyl]-1-[4-(trifluormethyl)phenyl]-3-azabicyclo[3.1.0]hexan;
(1S,5R)-3-{3-[(4,5-Dimethyl-1H-imidazol-2-yl)thio]propyl}-1-[4-(trifluormethyl)phenyl]-3-azabicyclo[3.1.0]hexan;
(1S,5R)-3-{3-[(5-Methyl-1,3,4-thiadiazol-2-yl)thio]propyl}-1-[4-(trifluormethyl)phenyl]-3-azabicyclo[3.1.0]hexan;
(1S,5R)-3-{3-[(5-Methyl-1,3,4-oxadiazol-2-yl)thio]propyl}-1-[4-(trifluormethyl)phenyl]-3-azabicyclo[3.1.0]hexan;
(1S,5R)-3-{3-[(4,5-Dimethyl-1,3-oxazol-2-yl)thio]propyl}-1-[4-(trifluormethyl)phenyl]-3-azabicyclo[3.1.0]hexan;
(1S,5R)-3-[3-(1,3-Oxazol-2-ylthio)propyl]-1-[4-(trifluormethyl)phenyl]-3-azabicyclo[3.1.0]hexan;
(1S,5R)-3-[3-(1,3-Thiazol-2-ylthio)propyl]-1-[4-(trifluormethyl)phenyl]-3-azabicyclo[3.1.0]hexan;
(1S,5R)-3-(3-{[5-(3-Pyridinyl)-1,3-thiazol-2-yl]thio}propyl)-1-[4-(trifluormethyl)phenyl]-3-azabicyclo[3.1.0]hexan;
(1S,5R)-3-(3-{[1-Methyl-5-(3-pyridinyl)-1H-imidazol-2-yl]thio}propyl)-1-[4-(trifluormethyl)phenyl]-3-azabicyclo[3.1.0]hexan;
(1S,5R)-3-{3-[(1-Methyl-5-phenyl-1H-imidazol-2-yl)thio]propyl}-1-[4-(trifluormethyl)phenyl]-3-azabicyclo[3.1.0]hexan;
(1S,5R)-3-{3-[(5-Phenyl-1,3-thiazol-2-yl)thio]propyl}-1-[4-(trifluormethyl)phenyl]-3-azabicyclo[3.1.0]hexan;
(1S,5R)-3-{3-[(5-Phenyl-1,3,4-thiadiazol-2-yl)thio]propyl}-1-[4-(trifluormethyl)phenyl]-3-azabicyclo[3.1.0]hexan;
(1S,5R)-3-(3-{[5-(5-Chlor-2-thienyl)-1,3,4-thiadiazol-2-yl]thio}propyl)-1-[4-(trifluormethyl)phenyl]-3-azabicyclo[3.1.0]hexan;
(1S,5R)-3-(3-{[5-(2-Pyridinyl)-1,3,4-oxadiazol-2-yl]thio}propyl)-1-[4-(trifluormethyl)phenyl]-3-azabicyclo[3.1.0]hexan;
(1S,5R)-3-(3-{[5-(4-Chlorphenyl)-1-methyl-1H-imidazol-2-yl]thio}propyl)-1-[4-(trifluormethyl)phenyl]-3-azabicyclo[3.1.0]hexan;
(1S,5R)-3-(3-{[5-(2-Furanyl)-1,3,4-oxadiazol-2-yl]thio}propyl)-1-[4-(trifluormethyl)phenyl]-3-azabicyclo[3.1.0]hexan;
(1S,5R)-3-(3-{[5-(4-Chlorphenyl)-1,3,4-oxadiazol-2-yl]thio}propyl)-1-[4-(trifluormethyl)phenyl]-3-azabicyclo[3.1.0]hexan;
(1S,5R)-3-(3-{[4-(4-Chlorphenyl)-1,3-thiazol-2-yl]thio}propyl)-1-[4-(trifluormethyl)phenyl]-3-azabicyclo[3.1.0]hexan;
(1S,5R)-3-{3-[(4-Phenyl-1H-imidazol-2-yl)thio]propyl}-1-[4-(trifluormethyl)phenyl]-3-azabicyclo[3.1.0]hexan;
oder ein Salz davon.

**11.** Ein Verfahren zur Herstellung einer Verbindung wie in Anspruch 1 definiert, wobei das Verfahren, für eine Verbindung der Formel (I), wobei A gleich S ist, Umsetzen einer Verbindung der Formel (V):

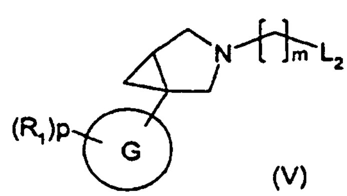

(V)

wobei $R_1$, p, G und m wie in Anspruch 1 definiert sind und $L_2$ eine Abgangsgruppe ist, mit einer Verbindung der Formel (VI):

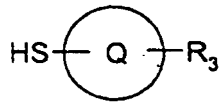

(VI)

wobei Q und $R_3$ wie in Anspruch 1 definiert sind;
und anschließend gegebenenfalls

(i) Entfernen jeglicher Schutzgruppe(n); und/oder
(ii) Bilden eines Salzes; und/oder
(iii) Umwandeln einer Verbindung wie in Anspruch 1 beansprucht in eine andere Verbindung wie in Anspruch 1 beansprucht

umfasst.

**12.** Verwendung einer Verbindung wie in einem der Ansprüche 1 bis 10 beansprucht bei der Herstellung eines Medikaments zur Behandlung von Psychose oder einem psychotischem Zustand, Suchtmittelmissbrauch, vorzeitiger Ejakulation oder kognitiver Beeinträchtigung bei einem Säuger.

**13.** Verwendung wie in Anspruch 12 beansprucht, wobei der Zustand Suchtmittelmissbrauch ist.

**14.** Verwendung wie in Anspruch 12 beansprucht, wobei der psychotische Zustand Schizophrenie ist.

**15.** Eine Verbindung wie in einem der Ansprüche 1 bis 10 beansprucht zur Verwendung bei der Therapie.

**16.** Eine Verbindung wie in einem der Ansprüche 1 bis 10 beansprucht zur Verwendung bei der Behandlung von Psychose oder einem psychotischen Zustand, von Suchtmittelmissbrauch, vorzeitiger Ejakulation oder kognitiver Beeinträchtigung.

**17.** Eine Verbindung wie in einem der Ansprüche 1 bis 10 beansprucht zur Verwendung bei der Behandlung von Schizophrenie.

**18.** Eine Verbindung wie in einem der Ansprüche 1 bis 10 beansprucht zur Verwendung bei der Behandlung von Suchtmittelmissbrauch.

**19.** Ein Arzneimittel, umfassend eine Verbindung wie in einem der Ansprüche 1 bis 10 beansprucht und einen pharmazeutisch verträglichen Träger.

## Revendications

**1.** Composé de formule (I) ou un de ses sels :

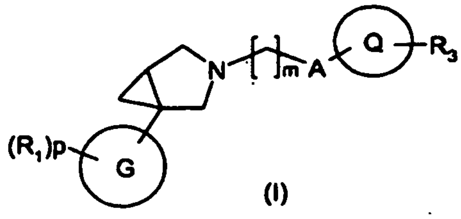

(I)

formule dans laquelle

- G est choisi dans le groupe consistant en des groupes : phényle, pyridyle, benzothiazolyle et indazolyle ;
- p est égal à 0, 1, 2, 3, 4 ou 5 ;
- $R_1$ est choisi indépendamment dans un groupe consistant en des groupes : halogéno, hydroxy, cyano, alkyle en $C_1$ à $C_4$, halogénalkyle en $C_1$ à $C_4$, alkoxy en $C_1$ à $C_4$, halogénalkoxy en $C_1$ à $C_4$, alcanoyle en $C_1$ à $C_4$ et $SF_5$ ; ou bien correspond à un groupe $R_2$ ;
- $R_2$ est choisi dans un groupe consistant en des groupes : isoxazolyle, $-CH_2$-N-pyrrolyle, 1,1-dioxydo-2-isothiazolidinyle, thiényle, thiazolyle, pyridyle et 2-pyrrolidinonyle, et un tel groupe est facultativement substitué avec un ou deux substituants choisis dans un groupe consistant en des substituants : halogéno, cyano, alkyle en $C_1$ à $C_4$, halogénalkyle en $C_1$ à $C_4$, alkoxy en $C_1$ à $C_4$ et alcanoyle en $C_1$ à $C_4$ ;
- m est égal à 2, 3, 4 ou 5 ;
- A représente S, O ou un groupe $-CH_2-$ ;
- Q représente un groupe hétéroaromatique pentagonal autre qu'un groupe triazolyle, groupe hétéroaromatique pentagonal qui est facultativement substitué avec un ou deux substituants alkyle en $C_1$ à $C_4$ ; et
- $R_3$ représente un atome d'hydrogène, un groupe alkyle en $C_1$ à $C_4$, phényle, hétérocyclyle, un groupe hétéroaromatique penta- ou hexagonal ou un groupe bicyclique octo- à undécagonal, n'importe lequel de ces groupes étant facultativement substitué avec 1, 2, 3 ou 4 substituants choisis dans le groupe consistant en des substituants : halogéno, cyano, alkyle en $C_1$ à $C_4$, halogénalkyle en $C_1$ à $C_4$, alkoxy en $C_1$ à $C_4$, alcanoyle en $C_1$ à $C_4$ et $SF_5$ ;

et, lorsque $R_1$ correspond à $R_2$, p est égal à 1.

**2.** Composé suivant la revendication 1, dans lequel G représente un groupe phényle.

**3.** Composé suivant la revendication 1, dans lequel $R_1$ représente un groupe trifluorométhyle.

**4.** Composé suivant l'une quelconque des revendications 1 à 3, dans lequel A représente un atome de soufre.

**5.** Composé suivant l'une quelconque des revendications 1 à 4, dans lequel m est égal à 3.

**6.** Composé suivant la revendication 1, répondant à la formule générale (IC), ou un de ses sels :

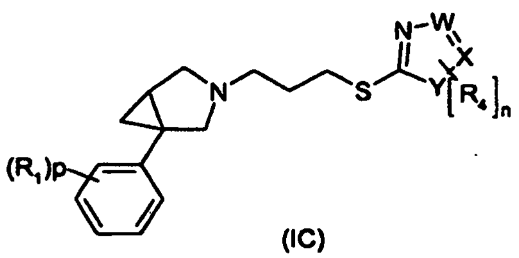

(IC)

dans laquelle $R_1$ et p sont tels que définis pour la formule (I), n est égal à 0, 1 ou 2, $R_4$ représente un atome d'hydrogène ou un groupe alkyle en $C_1$ à $C_4$, W représente C ou N, X représente N ou un groupe $CR_3$ et Y représente O, S, un groupe NH ou N-alkyle en $C_1$ à $C_4$, où $R_3$ représente un atome d'hydrogène, un groupe alkyle en $C_1$ à $C_4$, phényle, hétérocyclyle, un groupe hétéroaromatique penta- ou hexagonal ou un groupe bicyclique octo- à undécagonal, n'importe lequel de ces groupes étant facultativement substitué avec 1, 2, 3 ou 4 substituants choisis dans le groupe consistant en des substituants : halogéno, cyano, alkyle en $C_1$ à $C_4$, halogénalkyle en $C_1$ à $C_4$, alkoxy en $C_1$ à $C_4$, alcanoyle en $C_1$ à $C_4$ et $SF_5$.

**7.** Composé suivant l'une quelconque des revendications 1 à 6, dans lequel Q représente un groupe imidazolyle, thiadiazolyle, oxadiazolyle, thiazolyle, oxazolyle, tétrazolyle ou thiényle, chacun de ces groupes étant facultativement substitué avec un groupe alkyle en $C_1$ à $C_4$.

**8.** Composé suivant la revendication 1, répondant à la formule générale (ID), ou un de ses sels :

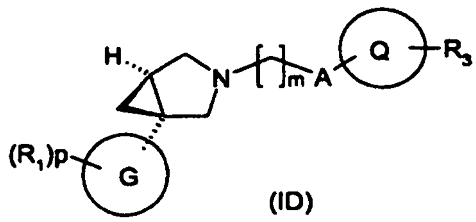

(ID)

dans laquelle G, p, $R_1$, m, A et $R_3$ sont tels que définis dans la revendication 1.

**9.** Composé suivant la revendication 1, répondant à la formule générale (IG), ou un de ses sels :

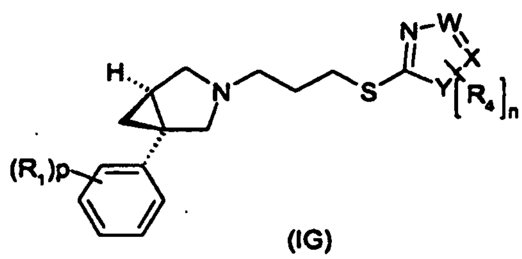

(IG)

dans laquelle $R_1$ et p sont tels que définis pour la formule (I), n est égal à 0, 1 ou 2, $R_4$ représente un atome d'hydrogène ou un groupe alkyle en $C_1$ à $C_4$, W représente C ou N, X représente N ou un groupe $CR_3$ et Y représente O, S, un groupe NH ou N-alkyle en $C_1$ à $C_4$, où $R_3$ représente un atome d'hydrogène, un groupe alkyle en $C_1$ à $C_4$, phényle, hétérocyclyle, un groupe hétéroaromatique penta- ou hexagonal ou un groupe bicyclique octo- à undécagonal, n'importe lequel de ces groupes étant facultativement substitué avec 1, 2, 3 ou 4 substituants choisis dans le groupe consistant en des substituants : halogéno, cyano, alkyle en $C_1$ à $C_4$, halogénalkyle en $C_1$ à $C_4$, alkoxy en $C_1$ à $C_4$, alcanoyle en $C_1$ à $C_4$ et $SF_5$.

**10.** Composé suivant la revendication 1, qui est :

le (1S,5R)-3-{3-[(1-méthyl-1H-tétrazole-5-yl)thio]propyl}-1-[4-(trifluorométhyl)phényl]-3-azabicyclo[3.1.0]hexane ;
le (1S,5R)-3-{3-[(1-méthyl-1H-imidazole-2-yl)thio]propyl}-1-[4-(trifluorométhyl)phényl]-3-azabicyclo[3.1.0]hexane ;
le (1S,5R)-3-[3-(1H-imidazole-2-ylthio)propyl]-1-[4-(trifluorométhyl)phényl]-3-azobicyclo[3.1.0]hexane ;
le (1S,5R)-3-{3-[(4,5-diméthyl-1H-imidazole-2-yl)thio]-propyl}-1-[4-(trifluorométhyl)phényl]-3-azabicyclo[3.1.0]hexane ;
le (1S,5R)-3-{3-[(5-méthyl-1,3,4-thiadiazole-2-yl)thio]-propyl}-1-[4-(trifluorométhyl)phényl]-3-azabicyclo[3.1.0]hexane ;
le (1S,5R)-3-{3-[(5-méthyl-1,3,4-oxadiazole-2-yl)thio]-propyl}-1-[4-(trifluorométhyl)phényl]-3-azabicyclo[3.1.0]hexane ;
le (1S,5R)-3-{3-[(4,5-diméthyl-1,3-oxadiazole-2-yl)thio]-propyl}-1-[4-(trifluorométhyl)phényl]-3-azabicyclo[3.1.0]hexane ;
le (1S,5R)-3-[3-(1,3-oxazole-2-ylthio)propyl]-1-[4-(trifluorométhyl)phényl]-3-azabicyclo[3.1.0]hexane ;
le (1S,5R)-3-[3-(1,3-thiazole-2-ylthio)propyl]-1-[4-(trifluorométhyl)phényl]-3-azabicyclo[3.1.0]hexane ;
le (1S,5R)-3-(3-{[5-(3-pyridinyl)-1,3-thiazole-2-yl]thio}-propyl)-1-[4-(trifluorométhyl)phényl]-3-azabicyclo[3.1.0]hexane ;
le (1S,5R)-3-(3-{[1-méthyl-5-(3-pyridinyl)-1H-imidazole-2-yl]thio}propyl)-1-[4-(trifluorométhyl)phényl]-3-azabicyclo-[3.1.0]hexane ;
le (1S,5R)-3-{3-[(1-méthyl-5-phényl-1H-imidazole-2-yl)-thio]propyl}-1-[4-(trifluorométhyl)phényl]-3-azabicyclo[3.1.0]-hexane ;
le (1S,5R)-3-{3-[(5-phényl-1,3-thiazole-2-yl)thio]propyl}-1-[4-(trifluorométhyl)phényl]-3-azabicyclo[3.1.0]hexane ;
le (1S,5R)-3-{3-[(5-phényl-1,3,4-thiadiazole-2-yl)thio]-propyl}-1-[4-(trifluorométhyl)phényl]-3-azabicyclo[3.1.0]hexane ;

le (1S,5R)-3-(3-{[5-(5-chloro-2-thiényl)-1,3,4-thiadiazole-2-yl]thio}propyl)-1-[4-(trifluorométhyl)phényl]-3-azabicyclo-[3.1.0]hexane ;
le (1S,5R)-3-(3-{[5-(2-pyridinyl)-1,3,4-oxadiazole-2-yl]thio}propyl)-1-[4-(trifluorométhyl)phényl]-3-azabicyclo-[3.1.0]hexane ;
le (1S,5R)-3-(3-{[5-(4-chlorophényl)-1-méthyl-1H-imidazole-2-yl]thio}propyl)-1-[4-(trifluorométhyl)phényl]-3-azabicyclo-[3.1.0]hexane ;
le (1S,5R)-3-(3-{[5-(2-furannyl)-1,3,4-oxadiazole-2-yl]-thio}propyl)-1-[4-(trifluorométhyl)phényl]-3-azabicyclo [3.1.0]-hexane ;
le (1S,5R)-3-(3-{[5-(4-chlorophényl)-1,3,4-oxadiazole-2-yl]thio}propyl)-1-[4-(trifluorométhyl)phényl]-3-azabicyclo-[3.1.0]hexane ;
le (1S,5R)-3-(3-{[4-(4-chlorophényl)-1,3-thiazole-2-yl]-thio}propyl)-1-[4-(trifluorométhyl)phényl]-3-azabicyclo [3.1.0]-hexane ;
le (1S,5R)-3-{3-[(4-phényl-1H-imidazole-2-yl)thio]propyl}-1-[4-(trifluorométhyl)phényl]-3-azabicyclo[3.1.0] hexane ;
ou un de ses sels.

**11.** Procédé pour la préparation d'un composé tel que défini dans la revendication 1, le procédé comprenant, pour un composé de formule (I) dans laquelle A représente S, la réaction d'un composé de formule (V) :

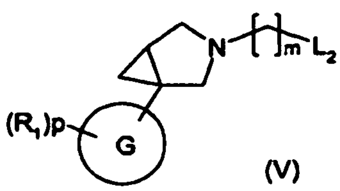

(V)

dans laquelle $R_1$, p, G et m sont tels que définis dans la revendication 1 et $L_2$ représente un groupe partant, avec un composé de formule (VI) :

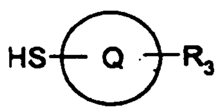

(VI)

dans laquelle Q et $R_3$ sont tels que définis dans la revendication 1 ;
et ensuite, facultativement

(i) l'élimination d'un ou plusieurs groupes protecteurs quelconques ; et/ou
(ii) la formation d'un sel ; et/ou
(iii) la conversion d'un composé suivant la revendication 1 en un autre composé suivant la revendication 1.

**12.** Utilisation d'un composé suivant l'une quelconque des revendications 1 à 10 dans la production d' un médicament destiné au traitement de la psychose ou d'un état psychotique, de l'abus d'une substance, de l'éjaculation précoce ou de l'altération cognitive chez un mammifère.

**13.** Utilisation suivant la revendication 12, dans laquelle l'affection est l'abus d'une substance.

**14.** Utilisation suivant la revendication 12, dans laquelle l'état psychotique est la schizophrénie.

**15.** Composé suivant l'une quelconque des revendications 1 à 10, destiné à être utilisé en thérapie.

**16.** Composé suivant l'une quelconque des revendications 1 à 10, destiné à être utilisé dans le traitement de la psychose ou d'un état psychotique, de l'abus d'une substance, de l'éjaculation précoce ou de l'altération cognitive.

**17.** Composé suivant l'une quelconque des revendications 1 à 10, destiné à être utilisé dans le traitement de la schizophrénie.

**18.** Composé suivant l'une quelconque des revendications 1 à 10, destiné à être utilisé dans le traitement de l'abus d'une substance.

**19.** Composition pharmaceutique comprenant un composé suivant l'une quelconque des revendications 1 à 10 et un support pharmaceutiquement acceptable.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 200240471 A, SmithKline Beecham
- WO 2005080382 A
- US 3505350 A
- EP 263066 A


### Non-patent literature cited in the description


- *J. Med Chem,* 1981, vol. 24 (5), 481-90
- **Shah RD et al.** *Curr Opin Drug Disc Dev,* 2001, vol. 4, 764-774
- **Freedman TB et al.** *Helv Chim Acta,* 2002, vol. 85, 1160-1165
- **Dyatkin AB et al.** *Chirality,* 2002, vol. 14, 215-219
- **Solladie -Cavallo A ; Balaz M et al.** *Tetrahedron Assym,* 2001, vol. 12, 2605-2611
- **Nafie LA et al.** Circular dichroism, principles and applications. John Wiley & Sons, 2000, 97-131
- **Nafie LA et al.** Infrared and Raman spectroscopy of biological materials. Marcel Dekker, 2001, 15-54
- **Polavarapu PL et al.** *J Anal Chem,* 2000, vol. 366, 727-734
- **Stephens PJ et al.** *Chirality,* 2000, vol. 12, 172-179
- **Solladie' -Cavallo A et al.** *Eur J Org Chem,* 2002, 1788-1796
- **Eliel EL ; Wilen SH.** Stereochemistry of organic compounds. John Wiley & Sons, 1994
- **T.W. Greene ; P.G.M. Wuts.** Protective groups in organic synthesis. John Wiley & sons, 1991
- **P.J. Kocienski.** Protecting Groups. Georg Thieme Verlag, 1994
- *J. Med. Chem.,* 1981, vol. 24, 481-490
- *J. Am. Chem. Soc.,* 1955, vol. 77, 2313
- *Synlett,* 2002, vol. 5, 829-831
- **Cheng ; Prusoff.** *Biochem. Pharmacol.,* 1973, vol. 22, 3099
- **Sokoloff et al.** *Nature,* 1990, vol. 347, 146-151
- **Schwartz et al.** *Clinical Neuropharmacology,* 1993, vol. 16 (4), 295-314
- **Levant.** *Pharmacol. Rev.,* 1997, vol. 49, 231-252
- **Schwartz et al.** *Brain Res. Reviews,* 1998, vol. 26, 236-242
- *Chem. Ber.,* 1976, vol. 109, 139-153
- *Acta Chemica Scandinavica,* 1961, vol. 15, 1124-1129
- *J. Heteroc. Chem.,* 1995, 227-234
- *Acta Chemica Scandinavica,* 1969, vol. 23 (8), 2879-87